# EUROPÄISCHE PATENTSCHRIFT

(11) **EP 3 178 545 B1**
(45) Veröffentlichungstag und Bekanntmachung des Hinweises auf die Patenterteilung: **20.06.2018**
(21) Anmeldenummer: 16202439.2
(22) Anmeldetag: 06.12.2016
(51) Int. Cl.: B01F 11/00, B01F 15/02, B01F 13/06, B01F 13/00, B01F 15/00

(54) **VAKUUMMISCHVORRICHTUNG MIT BEDIENELEMENT UND PUMPE ZUM MISCHEN VON POLYMETHYLMETHACRYLAT-KNOCHENZEMENT, UND VERFAHREN**
VACUUM MIXING DEVICE WITH OPERATING ELEMENT AND PUMP FOR MIXING POLYMETHYLMETHACRYLATE BONE CEMENT, AND METHOD
DISPOSITIF DE MÉLANGE SOUS VIDE AVEC ORGANE DE COMMANDE ET POMPE POUR MÉLANGER DU CIMENT OSSEUX EN POLYMÉTHACRYLATE DE MÉTHYLE, ET PROCÉDÉ

(30) Priorität: 07.12.2015 DE 102015121274
(43) Veröffentlichungstag der Anmeldung: 14.06.2017
(73) Patentinhaber: Heraeus Medical GmbH, 61273 Wehrheim (DE)
(72) Erfinder: Kluge, Thomas, 56179 Vallendar (DE); Vogt, Sebastian, 99092 Erfurt (DE)
(74) Vertreter: Schultheiss & Sterzel Patentanwälte PartG mbB

(56) Entgegenhaltungen:
- DE-B3-102014 108 569
- DE-U1- 20 005 333
- US-A- 5 505 538

## Beschreibung

Die Erfindung betrifft eine Vakuummischvorrichtung zum Mischen von Polymethylmethacrylat-Knochenzement (PMMA-Knochenzement) aus zwei Ausgangskomponenten, insbesondere zum Mischen eines medizinischen Knochenzements, und zur Lagerung der Ausgangskomponenten. Die Erfindung betrifft ferner ein Verfahren zum Mischen von Polymethylmethacrylat-Knochenzement.

Gegenstand der Erfindung ist somit eine Vakuummischvorrichtung für die Lagerung, Vermischung und gegebenenfalls auch den Austrag von Polymethylmethacrylat-Knochenzement. Weiterhin betrifft die Erfindung ein Verfahren zum Transfer von Monomerflüssigkeit in die Vakuummischvorrichtung und ein Verfahren zum Vermischen der Komponenten von Polymethylmethacrlyat-Knochenzement unter Vakuum beziehungsweise bei Unterdruck.

Polymethylmethacrylat-Knochenzemente (PMMA-Knochenzemente) gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind. Zudem weist das Knochenzementpulver einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine mechanische Destabilisierung des Knochenzements verursachen können.

Aus diesem Grund wird das Vermischen von Knochenzementpulver mit der Monomerflüssigkeit in Mischvorrichtungen mit Vakuumquellen bevorzugt, weil durch Mischen im Vakuum Lufteinschlüsse aus dem Zementteig weitgehend entfernt werden und damit eine optimale Zementqualität erreicht wird. Im Vakuum, beziehungsweise bei Unterdruck gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, WO 00/35506 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Auch bei diesen Mischsystemen ist eine externe Vakuumquelle notwendig. Das Patent DE 10 2009 031 178 B3 offenbart dabei eine Vakuum-Mischvorrichtung mit einem zweiteiligen Austragskolben, der auch für eine erfindungsgemäße Vakuummischvorrichtung einsetzbar ist. Dabei wird eine Kombination aus einem Gas-durchlässigen Sterilisationskolben und einem Gas-undurchlässigen Dichtungskolben verwendet. Auch die DE 200 05 333 U1 offenbart eine Vakuummischvorrichtung und ein Verfahren zur Vermischung von PMMA-Knochenzement.

Bei der Verwendung von Vakuummischvorrichtungen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischvorrichtungen notwendig. Diese Vakuumschläuche müssen den Vakuummischvorrichtungen beigelegt sein. Vor dem Mischen mit einer Vakuummischvorrichtung muss daher zuerst die Vakuumpumpe im Operations-Saal (OP-Saal) aufgebaut und an eine Energiequelle, wie beispielsweise Druckluft, oder an eine elektrische Spannungsquelle angeschlossen werden. Danach wird die Vakuumpumpe mit einem Vakuumschlauch mit der Vakuummischvorrichtung verbunden. Diese Montageschritte kosten wertvolle OP-Zeit und sind potentiell fehlerbehaftet. Die Vakuumpumpe und die Verbindungsleitungen zur Vakuummischvorrichtung und zu externen Energiequellen und Versorgungsleitungen benötigen Platz und stellen potentielle Stolperfallen und Hindernisse dar, die den gelegentlich hektischen Ablauf während einer Operation stören können.

Ein weiteres interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für einen Mischstab oder ein Mischrohr nicht ohne weiteres dauerhaft vakuumdicht ist.

Die Aufgabe der Erfindung besteht also darin, die Nachteile des Stands der Technik zu überwinden. Insbesondere sollen die Nachteile der bekannten Vakuummischvorrichtungen als Full-Prepacked-Systeme mit externer Vakuumquelle überwunden werden, ohne dass ein Unterdruck über lange Zeit gehalten werden muss. Die Erfindung hat insbesondere die Aufgabe eine Vakuummischvorrichtung zu entwickeln, bei der erst unmittelbar vor dem Vermischen der Zementkomponenten ein Unterdruck erzeugt wird. Die Vorrichtung soll maximal vereinfacht sein und es erlauben, zumindest einmalig einen Unterdruck in einer Zementkartusche gegenüber der umgebenden Atmosphäre zu erzeugen. Weiterhin kann es vorteilhaft sein, wenn die Vakuummischvorrichtung in der Lage ist, einen Transfer von Monomerflüssigkeit aus einem Monomerbehälter in eine mit Zementpulver gefüllte Kartusche zu ermöglichen. Es soll dann ferner ein Verfahren bereitgestellt werden, das einen Monomertransfer und ein Vakuummischen in Full-Prepacked-Mischvorrichtungen ermöglicht. Weiterhin soll die zu entwickelnde Vakuummischvorrichtung hauptsächlich aus kostengünstigem Kunststoff gefertigt werden können.

Die Erfindung hat ferner die Aufgabe, eine einfache geschlossene Prepack-Mischvorrichtung zu entwickeln, bei der Polymethylmethacrylat-Knochenzementpulver und Monomerflüssigkeit in separaten Kompartimenten gelagert und anschließend vermischt werden können. Unmittelbar vor der Vermischung der Komponenten soll die Monomerflüssigkeit ohne Nutzung von externen Vakuumquellen, externen elektrischen Antrieben und externen Druckluftantieben in das Zementpulver transferiert werden. Mit der Prepack-Mischvorrichtung soll unabhängig von zusätzlichen externen Vorrichtungen rein durch manuelle Betätigung Polymethylmethacrylat-Knochenzement erzeugt werden können. Dabei soll die manuelle Betätigung so weit wie möglich vereinfacht werden. Vorzugsweise soll das Öffnen der Monomerampulle, beziehungsweise der Monomerampullen, der Monomertransfer, die Erzeugung eines Vakuums oder eines Unterdrucks und die Vermischung der Zementkomponenten nur durch möglichst eine einfache Bewegung bewirkt werden, die besonders bevorzugt nur wenige Male, beispielsweise 3 bis 5 mal, wiederholt werden muss. Dadurch soll die Benutzung der Vakuummischvorrichtung für den Anwender maximal vereinfacht werden, so dass kostenintensive Schulungen eingeschränkt beziehungsweise eingespart werden können. Weiterhin soll durch eine maximal vereinfachte Bedienung der Vakuummischvorrichtung mögliche Bedienungsfehler minimiert werden, wodurch die Patientensicherheit erhöht wird.

Vom medizinischen Anwender soll das Polymethylmethacrylat-Knochenzementpulver mit der Monomerflüssigkeit innerhalb der Vakuummischvorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit muss ausgeschlossen sein. Bei der zu entwickelnden Vakuummischvorrichtung handelt es sich um eine Full-Prepack Vakuummischvorrichtung. Die Vakuummischvorrichtung soll so beschaffen sein, dass ein Transfer der Monomerflüssigkeit in das Polymethylmethacrylat-Knochenzementpulver durch Vakuum ohne die Verwendung von externen Vakuumpumpen erfolgt. Des Weiteren soll die Vakuummischvorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleisten. Besonders bevorzugt soll die Vakuummischvorrichtung möglichst weitgehend auch ohne interne Energiespeicher, wie beispielsweise Batterien oder auch mechanische Energiespeicher auskommen Die Vakuummischvorrichtung soll ohne zusätzliche technische Ausrüstung autonom verwendungsfähig sein.

Des Weiteren soll eine kostengünstig zu fertigende und zuverlässig funktionierende Vakuummischvorrichtung zum Mischen eines medizinischen Zements und gegebenenfalls zur Lagerung der Ausgangskomponenten sowie ein Verfahren zum Mischen des Knochenzements gefunden werden, bei dem eine möglichst einfache manuelle Bedienung zum Mischen der Ausgangskomponenten angewendet werden kann, möglichst ohne dass Lufteinschlüsse in dem Mischgut entstehen können.

Die Hauptkomponente des Polymethylmethacrylat-Knochenzements als Mischgut soll ein Pulver sein und die zweite Komponente soll in Form einer Flüssigkeit vorliegen. Die beiden Ausgangskomponenten des Knochenzements sollen bevorzugt in der Vakuummischvorrichtung getrennt gelagert werden können und durch Anwendung der Vorrichtung sicher zusammengeführt werden können.

Die Aufgaben der Erfindung werden gelöst durch eine Vakuummischvorrichtung zum Mischen von Polymethylmethacrylat-Knochenzement aus einer Monomerflüssigkeit und einem Zementpulver, die Vakuummischvorrichtung aufweisend zumindest eine Kartusche umfassend einen evakuierbaren Innenraum zum Mischen des Knochenzements,
eine Mischeinrichtung zum Durchmischen des Inhalts des Innenraums der zumindest einen Kartusche, die beweglich im Innenraum angeordnet ist,
eine Aufnahme zur Aufnahme eines die Monomerflüssigkeit enthaltenden separaten Behälters oder aufweisend einen integrierten Behälter enthaltend die Monomerflüssigkeit,
eine Öffnungseinrichtung, die im Bereich der Aufnahme gegen die Aufnahme beweglich angeordnet ist, so dass durch eine Bewegung der Öffnungseinrichtung ein in der Aufnahme angeordneter separater Behälter mit der Öffnungseinrichtung zu öffnen ist, oder die Öffnungseinrichtung im Bereich des integrierten Behälters gegen den integrierten Behälter beweglich angeordnet ist, so dass durch eine Bewegung der Öffnungseinrichtung der integrierte Behälter mit der Öffnungseinrichtung zu öffnen ist,
eine Pumpe, in der ein beweglicher Kolben zum Erzeugen eines Unterdrucks angeordnet ist und der einen Pumpraum der Pumpe begrenzt, und
eine Verbindungsleitung, die den Innenraum der zumindest einen Kartusche mit dem Pumpraum der Pumpe verbindet, wobei
die Vakuummischvorrichtung ein von außen bedienbares Bedienelement aufweist, wobei mit dem Bedienelement der Kolben in der Pumpe manuell bewegbar ist, und wobei
mit demselben Bedienelement die Öffnungseinrichtung gegen die Aufnahme oder gegen den integrierten Behälter zu bewegen ist, und
mit demselben Bedienelement die Mischeinrichtung im Innenraum der Kartusche zum Durchmischen des Inhalts des Innenraums der Kartusche bewegbar ist.

Bevorzugt ist die Vakuummischvorrichtung auch zum Lagern der Ausgangskomponenten des Polymethylmethacrylat-Knochenzements geeignet. Besonders bevorzugt sind die Monomerflüssigkeit und/oder das Zementpulver in der Vakuummischvorrichtung enthalten. Bei den Ausgangskomponenten des Polymethylmethacrylat-Knochenzements handelt es sich um das Zementpulver und die Monomerflüssigkeit, wobei die Monomerflüssigkeit bevorzugt in einer Glasampulle enthalten ist, die als separater Behälter in der Aufnahme angeordnet ist. Die Monomerflüssigkeit kann aber auch in einem Folienbeutel als separater Behälter enthalten sein oder sie kann in dem integrierten Behälter enthalten sein, der durch die Aufnahme beziehungsweise die Vakuummischvorrichtung selbst gebildet wird.

Der Begriff "Vakuummischvorrichtung" soll nicht in dahingehend falsch verstanden werden, dass ein Vakuum mit irgendetwas gemischt wird, sondern dass die Ausgangskomponenten des Knochenzements, also die Monomerflüssigkeit und das Zementpulver, unter Vakuum oder unter einem Druck kleiner als der Umgebungsdruck (Unterdruck) gemischt werden können.

Der Begriff Unterdruck bezieht sich vorliegend immer auf einen relativ zur umgebenden Atmosphäre bezogenen Druck, der also kleiner ist als der umgebende Atmosphärendruck.

Aufgrund der speziellen Anforderungen, wie die des geringen Volumens des Innenraums der Kartusche, sind aufwendigere Pumpensysteme nicht notwendig.

Bevorzugt kann vorgesehen sein, dass die Pumpe in die Vakuummischvorrichtung integriert ist. Bevorzugt kann ferner vorgesehen sein, dass der Druck in dem Innenraum der zumindest einen Kartusche durch den Pumpvorgang um wenigstens 50% reduzierbar ist, bevorzugt um wenigstens 90% reduzierbar ist.

Bevorzugt kann erfindungsgemäß auch vorgesehen sein, dass das Zementpulver in dem Innenraum der Kartusche enthalten ist. Das Zementpulver muss dann nicht in den Innenraum der Kartusche eingefüllt werden.

Durch die Kopplung der Öffnungseinrichtung mit dem Bedienelement ist der Behälter in der Aufnahme oder ist der integrierte Behälter durch manuelles Bedienen des Bedienelements mit der Öffnungseinrichtung zu öffnen.

Evakuierbar bedeutet im Zusammenhang mit der vorliegenden Erfindung, dass ein Gas entfernbar ist, also beispielsweise ein Gas aus dem Innenraum der Kartusche abgesaugt werden kann, so dass anschließend im Innenraum der Kartusche ein Unterdruck verbleibt. Dieser Unterdruck kann zum Einsaugen der Monomerflüssigkeit genutzt werden.

Die Vakuummischvorrichtung umfasst entweder eine Aufnahme, in die ein separater Behälter, wie beispielsweise eine Glasampulle oder ein Folienbeutel, die oder der die Monomerflüssigkeit enthält, eingesetzt werden kann, oder einen integrierten Behälter, der als integraler Teil der Vakuummischvorrichtung ausgebildet ist und in dem die Monomerflüssigkeit bereits enthalten ist.

Es kann auch vorgesehen sein, dass mehr als eine Kartusche mit jeweils einem Innenraum vorgesehen ist, wobei dann in jedem Innenraum eine Mischeinrichtung vorgesehen ist und jeder Innenraum über eine Verbindungsleitung mit dem Pumpraum der Pumpe oder mit jeweils einem Pumpraum einer Mehrzahl separater Pumpen verbunden ist.

Bevorzugt ist die Aufnahme auch zur Fixierung einer Glasampulle beziehungsweise der Glasampulle in der Aufnahme geeignet und vorgesehen. Die Glasampulle muss hierzu selbstverständlich passend geformt sein. Beispielsweise kann die Glasampulle in Presspassung in die Aufnahme eingesteckt werden.

Es kann erfindungsgemäß vorgesehen sein, dass die Aufnahme auf einer Seite mit einem Deckel verschlossen ist. Dabei kann bevorzugt in dem Deckel zumindest eine gasdurchlässige Öffnung vorgesehen sein, durch die Gas in die Aufnahme einströmen beziehungsweise nachströmen kann, wenn die Monomerflüssigkeit aus der Aufnahme herausgezogen wird. Hierdurch soll vermieden werden, dass sich in der Aufnahme ein Unterdruck bildet, der dem Fluss der Monomerflüssigkeit in den Innenraum der Kartusche entgegenwirkt.

Bevorzugt weist die Kartusche eine druckdichte Durchführung auf, durch die ein Stab, eine Kabel oder eine Mischwelle durchgeführt ist, mit dem die Mischeinrichtung von außerhalb der Kartusche zu bewegen ist. Dazu ist der Stab, das Kabel oder die Mischwelle bevorzugt drehbar und in Längsrichtung verschiebbar in der Durchführung gelagert. Mit der Mischeinrichtung kann der Inhalt der Kartusche gut durchmischt werden.

Bevorzugte Ausführungsformen können sich dadurch auszeichnen, dass die Vakuummischvorrichtung weniger als 10 kg Gesamtgewicht hat, besonders bevorzugt weniger als 2 kg Gesamtgewicht hat, besonders bevorzugt weniger als 1 kg.

Diese geringen Gewichte sind mit dem erfindungsgemäßen Aufbau der Vakuummischvorrichtung mit manuellem bedienbarem Bedienelement und der Pumpe möglich. Das geringe Gewicht hat den Vorteil, dass die Vakuummischvorrichtung mitgenommen werden kann, transportabel ist und ohne Anschluss an Versorgungsleitungen und ohne große Vorbereitungen einsetzbar ist.

Es kann erfindungsgemäß vorgesehen sein, dass ein Sieb und/oder ein Filter das, der oder die unterhalb der Aufnahme, des separaten Behälters oder unterhalb des integrierten Behälters angeordnet ist oder sind, so dass der Inhalt des geöffneten integrierten Behälters oder separaten Behälters durch das Sieb und/oder den Filter fließt.

Damit können Glassplitter, Folienschnipsel oder andere Rückstände des Verschlusses beziehungsweise des separaten oder integrierten Behälters, die beim Öffnen des separaten oder integrierten Behälters mit der Öffnungseinrichtung entstanden sind, zurückgehalten werden. Damit wird ein Verstopfen der Fluidverbindung zum Innenraum der Kartusche sowie eine Verunreinigung des herzustellenden Knochenzements verhindert.

Erfindungsgemäße Vakuummischvorrichtungen zeichnen sich dadurch aus, dass sie ohne einen elektrischen Antrieb auskommen. Es kann also erfindungsgemäß vorgesehen sein, dass die Vakuummischvorrichtung keinen elektrischen Antrieb aufweist oder zumindest die Pumpe, die Öffnungseinrichtung und die Mischeinrichtung nicht mit einem elektrischen Antrieb angetrieben werden. Stattdessen erfolgt der Antrieb dieser Bauteile erfindungsgemäß über das manuell bedienbare Bedienelement. Ebenso können erfindungsgemäße Vakuummischvorrichtungen ohne Elektronik beziehungsweise elektronische Bauteile aufgebaut werden. Eine erfindungsgemäße Vakuummischvorrichtung kann sich also auch dadurch auszeichnen, dass darin keine Elektronik verbaut ist oder dass zumindest zum Antrieb der Pumpe, der Öffnungseinrichtung und der Mischeinrichtung keine Elektronik oder keine elektronischen Bauteile angewendet werden. Elektromotoren oder Kompressoren werden also nicht zum Aufbau erfindungsgemäßer Vakuummischvorrichtungen benötigt.

Des Weiteren kann erfindungsgemäß vorgesehen sein, dass die Vakuummischvorrichtung keinen Energiespeicher aufweist, insbesondere keinen elektrischen Energiespeicher, wie beispielsweise eine Batterie oder einen Akkumulator, und keinen Druckgasspeicher, wie beispielsweise eine CO₂-Druckgaspatrone, oder dass zumindest kein Energiespeicher, vorzugsweise kein elektrischer Energiespeicher oder Druckgasspeicher, zum Antrieb der Pumpe, der Öffnungseinrichtung und der Mischeinrichtung verwendet werden. Bevorzugt weist die Vakuummischvorrichtung aber auch keine elastischen Energiespeicher, wie beispielsweise gespannte Federn, auf.

Bei erfindungsgemäßen Vakuummischvorrichtungen kann vorgesehen sein, dass das Bedienelement mit dem Kolben derart verbunden oder verbindbar ist, dass der Kolben in der Pumpe durch Bedienen des Bedienelements manuell bewegbar ist.

Hiermit wird erreicht, dass der Kolben, insbesondere unmittelbar, mit dem Bedienelement bewegt werden kann. Dabei kann vorgesehen sein, dass sich der Kolben erst nach einem ersten Bedienen des Bedienelements mit dem Bedienelement in der Art verbindet, dass der Kolben durch eine weitere Bedienung des Bedienelements in der Pumpe bewegt wird. Für die Ausführung des Bedienelements ist dabei ein Hebel besonders gut geeignet, der um eine Achse hin und her gezogen beziehungsweise gedrückt beziehungsweise geschwenkt werden kann, so dass nach einem ersten Bewegen des Hebels der separate Behälter in der Aufnahme geöffnet wird oder der integrierte Behälter geöffnet wird und sich der Kolben und/oder die Mischeinrichtung dabei mit dem Bedienelement derart verbindet oder verbinden, dass bei einer umgekehrten Bewegung des Hebels der Kolben in der Pumpe und/oder die Mischeinrichtung in dem Innenraum der Kartusche bewegt wird oder werden. Zudem sind mit einem Hebel problemlos große Kräfte manuell in die Vakuummischvorrichtung zu übertragen.

Des Weiteren kann vorgesehen sein, dass die Aufnahme zumindest bereichsweise geschlossene Seitenwände zur Aufnahme einer Glasampulle als separaten Behälter aufweist, wobei die Aufnahme zumindest eine verformbare geschlossene Seitenwand aufweist und gegenüber der verformbaren Seitenwand ein Stützelement vorgesehen ist, wobei die Öffnungseinrichtung über das Bedienelement gegen die verformbare Seitenwand der Aufnahme zu pressen ist, so dass sich die verformbare Seitenwand derart verformt, dass eine in der Aufnahme angeordnete passende Glasampulle mit der Öffnungseinrichtung aufzubrechen ist.

Durch diese Maßnahme kann die Aufnahme weitgehend nach außen geschlossen werden. Zudem kann hierdurch sichergestellt werden, dass eine Glasampulle auch innerhalb der geschlossenen Aufnahme geöffnet werden kann, ohne dass ohne weiteres Monomerflüssigkeit aus der Vakuummischvorrichtung austreten kann, wodurch die Gefahr einer Kontamination der Umgebung der Vakuummischvorrichtung mit dem Inhalt der Kartusche, insbesondere mit der Monomerflüssigkeit, ausgeschlossen werden kann oder die Gefahr hierfür zumindest stark reduziert wird.

Mit einer Weiterbildung der vorliegenden Erfindung wird vorgeschlagen, dass die Öffnungseinrichtung einen ersten Hebel aufweist, der um eine erste Achse drehbar gegen die Aufnahme oder den integrierten Behälter gelagert ist, wobei ein freies Ende des ersten Hebels gegen eine verformbare Seitenwand der Aufnahme oder den integrierten Behälter drückbar ist, wobei das Bedienelement durch einen zweiten Hebel gebildet ist, der um eine zweite Achse drehbar gegen die Aufnahme oder den integrierten Behälter gelagert ist, wobei die zweite Achse den zweiten Hebel in einen kurzen Hebelarm und einen langen Hebelarm teilt, wobei ein Ende des kurzen Hebelarms durch manuelles Bedienen des langen Hebelarms gegen den ersten Hebel zu drücken ist, so dass das freie Ende des ersten Hebels gegen die verformbare Seitenwand drückt und diese derart verformt, dass eine in der Aufnahme befindlicher separater Behälter zu öffnen ist, oder das freie Ende des ersten Hebels gegen den integrierten Behälter drückt, so dass der integrierte Behälter sich zu einer Fluidverbindung hin öffnet.

Dabei kann vorgesehen sein, dass der separate Behälter eine zur Aufnahme passende Glasampulle ist, die durch den Druck des freien Endes des ersten Hebels aufzubrechen ist oder ein in der Aufnahme angeordneter Folienbeutel ist, der durch den Druck des freien Endes des ersten Hebels aufzustechen, aufzuschlitzen oder aufzureißen ist.

Ferner kann bei solchen Ausführungen vorgesehen sein, dass am freien Ende des ersten Hebels auf der zur Aufnahme zugewandten Seite eine Kante angeordnet ist. Das Längenverhältnis des langen Hebelarms zum kurzen Hebelarm beträgt bevorzugt mindestens 5 zu 1. Des Weiteren kann vorgesehen sein, dass der zweite Hebel in der gleichen Ebene zu drehen ist, wie der erste Hebel, wobei die Bewegung des zweiten Hebels in die Bewegung des ersten Hebels eingreift. Bevorzugt kann auch vorgesehen sein, dass die zweite Achse des zweiten Hebels oberhalb der ersten Achse des ersten Hebels angeordnet ist, wobei vorzugsweise die erste Achse des ersten Hebels und die zweite Achse des zweiten Hebels parallel zueinander angeordnet sind.

Mit derartigen Vakuummischvorrichtungen mit Glasampulle gelingt es, eine Glasampulle großflächig innerhalb der Vorrichtung beziehungsweise der Zementiervorrichtung aufzubrechen, so dass die Monomerflüssigkeit aus der Glasampulle in kurzer Zeit ausströmt und zum Mischen mit dem medizinischen Knochenzementpulver bereitsteht. Mit Hilfe der beiden Hebel, die in Wechselwirkung miteinander stehen, ist es möglich, den Druck auf die Glasampulle in Richtung des Sitzes der Glasampulle in der Aufnahme zu richten, so dass ein Ausweichen der Glasampulle aus der Aufnahme heraus ausgeschlossen werden kann. Gleichzeitig kann ein sehr genau definierter lokaler Druck auf die Glasampulle ausgeübt werden, mit dem die Glasampulle in der Vakuummischvorrichtung aufgebrochen werden kann. Mit Hilfe der verformbaren Seitenwand kann dafür gesorgt werden, dass die Kraft durch diese Seitenwand ins Innere der Aufnahme auf die Glasampulle übertragen wird, wobei die Aufnahme dabei geschlossen bleibt. Ein Austreten der Monomerflüssigkeit aus der Aufnahme kann somit ausgeschlossen werden. Mit Hilfe des Siebs und/oder des Filters können Glassplitter, die beim Öffnen der Glasampulle entstehen können, zurückgehalten werden. Die Monomerflüssigkeit ist dann zum Mischen mit dem Knochenzementpulver nutzbar.

Der besondere Vorteil der erfindungsgemäßen Vorrichtung besteht auch darin, dass beliebige Glasampullen, unabhängig von der Ampullenlänge und der Geometrie des Ampullenkopfs, sicher geöffnet werden können, wenn der Ampullendurchmesser gleich oder etwas größer ist als der Innendurchmesser des Ampullenhalters beziehungsweise der Aufnahme. Weiterhin ist es ein besonderer Vorteil, dass beim Brechen der Ampullenwand im Bereich des Ampullenbodens die in der Glasampulle enthaltene Flüssigkeit unabhängig von der Oberflächenspannung sofort komplett ausfließt. Dagegen fließt bei konventionellen Ampullenbrechern die Flüssigkeit nach Abtrennen des Ampullenkopfs durch den relativ engen Querschnitt des Ampullenhalses deutlich langsamer aus. Dabei werden halbwegs große Auslaufgeschwindigkeiten nur erzielt, wenn entweder die Glasampulle im Winkel von ca. 45° mit dem Ampullenhals nach unten gehalten wird oder wenn der Querschnitt des Ampullenhalses so groß ist, dass die Oberflächenspannung der Flüssigkeit den Meniskus der Flüssigkeit nicht im Ampullenhals halten kann.

Bevorzugt ist die Aufnahme ein Hohlzylinder. Ebenfalls bevorzugt besteht die Aufnahme aus einem Elastomer oder umfasst einen Einsatz aus einem Elastomer, wie beispielsweise ein Ethylen-Propylen-Dien-Kautschuk (EPDM).

Ferner kann dabei vorgesehen sein, dass in der Aufnahme ein Absatz zur Auflage der Glasampulle angeordnet ist, wobei der Absatz kleiner als die Hälfte der Fläche des Ampullenbodens oder des Ampullenquerschnitts ist. Dabei kann wiederum bevorzugt vorgesehen sein, dass der Absatz derart in der Aufnahme angeordnet ist, dass der Abstand zwischen dem Absatz und einem darunter angeordneten Sieb und/oder Filter gleich oder größer ist als der Außendurchmesser der einzusetzenden Glasampulle.

Mit einer besonders einfach und kostengünstig zu realisierenden Vakuummischvorrichtung wird vorgeschlagen, dass das Bedienelement manuell beweglich ist, vorzugsweise ein um eine Achse schwenkbarer Hebel ist, wobei das Bedienelement derart mit der Öffnungseinrichtung, der Pumpe und der Mischeinrichtung in Wirkverbindung steht oder in Wirkverbindung zu bringen ist, dass bei einem ersten Bedienen des Bedienelements ein separater Behälter in der Aufnahme oder der integrierte Behälter zu öffnen ist und bei einem weiteren Bedienen des Bedienelements der Kolben in der Pumpe anzutreiben ist und die Mischeinrichtung im Innenraum anzutreiben ist.

Hiermit kann eine manuell aufzubringende Kraft, die auf das Bedienelement, insbesondere den Hebel, einwirkt, dazu verwendet werden, zunächst den separaten oder integrierten Behälter zu öffnen und anschließend die Pumpe und die Mischeinrichtung mit demselben Bedienelement anzutreiben beziehungsweise zu bewegen. Hebel als Bedienelemente sind für die Übertragung manueller Kraft in die Vakuummischvorrichtung besonders gut geeignet. Auch weil über die Länge des Hebelarms eine Verstärkung der nutzbaren Kraft möglich ist.

Dabei kann vorgesehen sein, dass der Kolben der Pumpe und/oder die Mischeinrichtung über ein flexibles Kabel und/oder eine Stange anzutreiben sind, wobei an dem flexiblen Kabel und/oder der Stange ein Rastmittel vorgesehen ist, das nach erstmaligem Bedienen des Bedienelements in ein Gegenrastmittel am Bedienelement oder in ein mit dem Bedienelement verbundenes Gegenrastmittel greift, so dass bei einem der Rastung nachfolgenden Bedienen des Bedienelements der Kolben der Pumpe und/oder die Mischeinrichtung über das Kabel und/oder die Stange mit dem Bedienelement anzutreiben sind.

Hiermit kann eine Übertragung der Kraft von dem Bedienelement auf den Kolben und/oder die Mischeinrichtung erfolgen. Die flexiblen Kabel sind besonders gut zur Übertragung der Kraft geeignet, da sich damit ohne aufwendige Mechanik die Richtung der Kraft umlenken lässt. Vorzugsweise ist das flexible Kabel ausreichend steif beziehungsweise starr, so dass sich der Kolben und/oder vor allem die Mischeinrichtung in beide Richtungen hin und her bewegen lässt. Hierzu kann das Kabel geführt und oder gestützt werden. Beispielsweise kann das Kabel hierzu in einem Kanal geführt sein oder es kann an geeigneten Stellen durch ein Gehäuse und durch Streben und/oder Umlenkrollen im Gehäuse gestützt beziehungsweise umgelenkt werden. Durch die Anwendung des Rastmittels und des Gegenrastmittels kann sichergestellt werden, dass zunächst der separate Behälter in der Aufnahme oder der integrierte Behälter geöffnet wird und die Monomerflüssigkeit vollständig ausläuft, bevor die Pumpe beziehungsweise der Kolben der Pumpe und/oder die Mischeinrichtung bedient wird. Damit kann beispielsweise verhindert werden, dass die Pumpe schon betrieben wird, bevor die Monomerflüssigkeit zur Verfügung steht.

Es wird ferner vorgeschlagen, dass die Mischeinrichtung durch Bedienen des Bedienelements in dem Innenraum axial in Längsrichtung beweglich ist.

Hiermit wird insbesondere für einen zylindrischen Innenraum eine sehr weitreichende Durchmischung des Innenraums erreicht. Gleichzeitig kann die Mischeinrichtung kompakt aufgebaut werden, da sie sich nicht entlang der gesamten Länge des Innenraums erstrecken muss.

Bevorzugt kann auch vorgesehen sein, dass die Mischeinrichtung durch Bedienen des Bedienelements in dem Innenraum um die Längsachse des Innenraums drehbar ist, wobei hierzu vorzugsweise sich ein mit der Mischeinrichtung verbundener Zylinder mit einem Außengewinde in einer feststehenden Hülse mit einem passenden Innengewinde bewegt, so dass bei einer Bewegung des Zylinders entlang der Längsrichtung innerhalb der Hülse eine Drehung des Zylinders erzwungen wird, wobei sich die Drehung des Zylinders auf die Mischeinrichtung im Innenraum der Kartusche überträgt.

Hierdurch wird eine noch stärkere Durchmischung des Inhalts des Innenraums erreicht. Auch an der Wand des Innenraums anliegender Knochenzement kann dadurch effektiv durchmischt werden. Der Knochenzement wird so schneller und effektiver durchmischt.

Mit einer Weiterbildung der vorliegenden Erfindung kann des Weiteren vorgesehen sein, dass der Pumpraum der Pumpe gasdicht ist und im Inneren der Pumpe angeordnet ist, wobei der Kolben über das Bedienelement manuell in zumindest einer Richtung antreibbar ist, so dass durch die Bewegung des Kolbens der Pumpraum zu vergrößern ist und mit dem dadurch im Pumpraum entstehenden Unterdruck der Innenraum der zumindest einen Kartusche durch die Verbindungsleitung evakuierbar ist.

Hiermit wird eine besonders gute Pumpwirkung der Pumpe erreicht und damit das in dem Innenraum der Kartusche erzeugbare Vakuum verbessert. Besonders bevorzugt lässt sich der Kolben mit dem Bedienelement manuell wiederholt in zwei Richtungen entlang der Achse des Pumpraums bewegen, so dass ein wiederholtes Pumpen zum Evakuieren des Innenraums der Kartusche möglich ist.

Dabei kann vorgesehen sein, dass die Volumenvergrößerung des Pumpraums mindestens so groß ist wie das freie Volumen des Innenraums der Kartusche, bevorzugt die Volumenvergrößerung des Pumpraums mindestens so groß ist wie die Summe des Volumens des Innenraums der Kartusche, in dem das Zementpulver des PMMA-Knochenzements enthalten ist, und des Volumens der Verbindungsleitung und des Volumens einer Fluidverbindung zwischen dem Innenraum der Kartusche und der Aufnahme für den separaten Behälter oder dem integrierten Behälter und des Volumens einer mit dem Zementpulver des PMMA-Knochenzements in der Kartusche zu mischenden Monomerflüssigkeit.

Hierdurch wird sichergestellt, dass die Pumpe den Innenraum der Kartusche bereits mit einem Hub oder mit wenigen Hüben des Kolbens evakuieren kann. Dabei ist das Volumen des Pumpraums vor dem Pumpvorgang idealerweise möglichst klein. Es kann also bevorzugt vorgesehen sein, dass das Volumen des Pumpraums nach dem Pumpvorgang zumindest 10 mal größer ist als das Volumen des Pumpraums vor dem Pumpvorgang, besonders bevorzugt zumindest 20 mal größer ist als das Volumen des Pumpraums vor dem Pumpvorgang. Bevorzugt liegt der Kolben nicht derart dicht an dem Innenraum der Pumpe, insbesondere der Deckfläche eines Hohlzylinders mit dem Anschluss für die Verbindungsleitung, flächenbündig an, dass sich der Kolben nicht an dieser Fläche festsaugt und zum Starten der Bewegung nur schwer bewegt werden kann. Hierzu können Abstandhalter an der Deckfläche im Pumpraum und/oder an dem Kolben im Pumpraum vorgesehen sein.

Um die Pumpe mit mehreren Bedienungen des Bedienelements bedienen zu können, beziehungsweise einen mehrfachen Hub des Kolbens der Pumpe nutzen zu können, kann vorgesehen sein, dass in der Verbindung vom Pumpraum zu der Verbindungsleitung oder in der Verbindungsleitung ein erstes Einwegventil angeordnet ist, das offen ist oder öffnet, wenn im Pumpraum ein geringerer Druck herrscht als im Innenraum der Kartusche, und geschlossen ist oder schließt, wenn im Pumpraum ein gleicher oder ein höherer Druck herrscht als im Innenraum der Kartusche, und bevorzugt ein zweites Einwegventil den Pumpraum mit der Umgebung der Pumpe verbindet, das offen ist oder öffnet, wenn im Pumpraum ein höherer Druck herrscht als in der Umgebung der Pumpe, und geschlossen ist oder schließt, wenn im Pumpraum ein gleicher oder ein geringerer Druck herrscht als in der Umgebung der Pumpe, wobei die Einwegventile bevorzugt Rückschlagventile sind.

Mit dem ersten Einwegventil wird erreicht, dass eine Strömung von dem Innenraum der Kartusche in den Pumpraum möglich ist, umgekehrte Strömungen aber weitgehend verhindert werden. Durch das zweite Einwegventil wird erreicht, dass ein Überdruck im Pumpraum über das zweite Einwegventil abgeblasen werden kann.

Hiermit kann mit einem wiederholten Pumpen durch mehrfache Bewegung des Kolbens aufgrund eines mehrfachen Bedienens des Bedienelements ein besonders gutes Vakuum beziehungsweise ein besonders guter Unterdruck im Innenraum der Kartusche erzeugt werden, der sich auch während dem Mischvorgang aufrechterhalten beziehungsweise noch verbessern lässt.

Des Weiteren kann vorgesehen sein, dass der Kolben in einem Hohlzylinder axial beweglich gelagert ist, wobei der Hohlzylinder auf einer ersten Seite geschlossen ist oder bis auf eine Durchführung für eine mit dem Bedienelement und dem Kolben verbundene Stange oder verbundenen Kabel geschlossen ist, insbesondere durch einen Verschluss geschlossen ist, wobei vorzugsweise ein Pumpraum in dem Hohlzylinder zwischen dem Kolben und der ersten geschlossenen Seite gebildet ist wobei besonders bevorzugt der Hohlzylinder auf einer zweiten Seite offen ist.

Bevorzugt haben der Pumpraum und der Kolben eine zylindrische Geometrie. Durch diese Maßnahmen und auch durch die zylindrische Geometrie wird eine besonders einfach und kostengünstig zu fertigende Pumpe vorgeschlagen, die leicht zu bedienen ist und die besonders unanfällig für Fehlfunktionen ist.

Es wird auch vorgeschlagen, dass der Kolben über eine Stange und/oder ein Kabel mit dem Bedienelement verbunden oder verbindbar ist und vorzugsweise der Kolben durch Bedienen des Bedienelements in der Pumpe zu bewegen ist.

Hierdurch wird eine besonders einfache Vakuummischvorrichtung bereitgestellt, bei der keine große Gefahr für mögliche Störungen besteht. Die direkte Verbindung des Bedienelements mit dem Kolben über das Kabel und/oder die Stange kann mit einem einteiligen Spritzgussteil aus Kunststoff realisiert werden. Alternativ kann auch eine Übersetzung beziehungsweise ein Getriebe vorgesehen sein, mit dem die Kraft, die auf das Bedienelement ausgeübt wird, zum Kolben übersetzt wird, um eine kräftigere Bewegung des Kolbens zu ermöglichen.

Bei einer Weiterentwicklung der Vakuummischvorrichtung kann vorgesehen sein, dass in dem Innenraum der Kartusche ein beweglicher Austragskolben zum Austragen des gemischten Knochenzements aus der Kartusche angeordnet ist, wobei der Austragskolben vorzugsweise lösbar arretiert oder arretierbar ist, um eine Bewegung des Austragskolbens unter Einwirkung des Unterdrucks zu verhindern.

Mit dem Austragskolben wird die Bedienung der Vakuummischvorrichtung vereinfacht.

Dabei kann vorgesehen sein, dass der Austragskolben einen Durchgang mit einer gasdurchlässige Porenscheibe aufweist, die für Zementpulver undurchlässig ist, wobei der Durchgang mit der Porenscheibe den Innenraum der Kartusche mit der Verbindungsleitung und/oder der Umgebung gasdurchlässig verbindet, wobei der Durchgang gasdicht verschließbar ist, vorzugsweise mit einem Dichtungskolben des Austragskolbens gasdicht verschließbar ist.

Mit der Porenscheibe kann sichergestellt werden, dass der Innenraum der Kartusche mit dem Zementpulver darin mit Hilfe eines Gases wie Ethylenoxid sterilisiert werden kann, ohne dass die Gefahr besteht, dass das Zementpulver aus dem Innenraum der Kartusche nach außen in die Umgebung gelangt.

Bevorzugt kann auch vorgesehen sein, dass die Kartusche eine mit Zementpulver gefüllte Zementkartusche ist und in der Aufnahme ein separater Behälter enthaltend eine Monomerflüssigkeit angeordnet ist oder in dem integrierten Behälter eine Monomerflüssigkeit enthalten ist, wobei vorzugsweise die Aufnahme oder der integrierte Behälter durch ein zu öffnendes Trennelement flüssigkeitsundurchlässig mit dem Innenraum der Zementkartusche verbunden ist und/oder der Innenraum der Zementkartusche mit der Pumpe gasdurchlässig verbunden ist oder verbindbar ist.

Hiermit wird erreicht, dass die Monomerflüssigkeit aus dem separaten oder integrierten Behälter mit der gleichen Pumpbewegung beziehungsweise dem gleichen Pumpvorgang in den Innenraum der Kartusche überführt werden kann, mit dem der Innenraum der Kartusche evakuiert wird. Das Vakuum beziehungsweise der Unterdruck, der mit der Pumpe manuell erzeugt wird, wird dabei gleichzeitig zum Einsaugen der Monomerflüssigkeit in die Kartusche genutzt. Insbesondere kann das zu öffnende Trennelement erfindungsgemäß mit dem Bedienelement geöffnet werden, vorzugsweise nachdem der integrierte Behälter oder der separate Behälter geöffnet ist.

Ferner kann vorgesehen sein, dass die Kartusche, die Pumpe und alle Leitungen sowie die Aufnahme oder der integrierte Behälter mit einem gemeinsamen Fußteil und/oder einem Gehäuse fest und/oder lösbar verbunden sind, wobei vorzugsweise die Pumpe und alle Leitungen sowie die Aufnahme oder der separate Behälter fest mit dem Fußteil und/oder dem Gehäuse verbunden sind und die Kartusche lösbar mit dem Fußteil und/oder dem Gehäuse verbunden ist.

Eine derartige Vakuummischvorrichtung kann einfach aufgestellt werden und ist leicht bedienbar. Die Anwendung der Vakuummischvorrichtung wird dadurch vereinfacht. Am Ort der Anwendung muss dann lediglich eine ebene Unterlage zum Aufstellen der Vakuummischvorrichtung vorhanden sein, was in den meisten OP-Bereichen kein Problem darstellt.

Gemäß einer Ausführungsform kann vorgesehen sein, dass der separate Behälter enthaltend die Monomerflüssigkeit ein Folienbeutel ist, der in der Aufnahme mit der Öffnungseinrichtung aufschneidbar oder aufreißbar ist, oder eine Glasampulle ist, die in der Aufnahme mit der Öffnungseinrichtung aufbrechbar ist.

Hierdurch können handelsübliche Verpackungen der Monomerflüssigkeit eingesetzt werden, ohne dass diese außerhalb der Vakuummischvorrichtung geöffnet werden müssen.

Es wird vorgeschlagen, dass die Aufnahme oder der integrierte Behälter über eine Fluidverbindung mit dem Innenraum der Kartusche verbunden oder verbindbar ist, wobei bevorzugt die Einmündung der Fluidverbindung in den Innenraum der Kartusche auf der gegenüberliegenden Seite der Einmündung der Verbindungsleitung zwischen dem Innenraum und dem Pumpraum angeordnet ist.

Hiermit wird erreicht, dass die Monomerflüssigkeit über eine eigene Leitung (die Fluidverbindung) in den Innenraum der Kartusche geleitet werden kann.

Dabei kann vorgesehen sein, dass in der Fluidverbindung eine Schlaufe angeordnet ist, so dass die aus dem geöffneten separaten Behälter oder dem integrierten Behälter auslaufende Monomerflüssigkeit nicht ohne Unterdruck im Innenraum der Kartusche in den Innenraum der Kartusche fließen kann, wobei vorzugsweise unterhalb der Aufnahme oder des integrierten Behälters ein Speichervolumen zur Aufnahme des Flüssigkeitsvolumens der Monomerflüssigkeit angeordnet ist.

Bei korrekter Aufstellung der Vakuummischvorrichtung kann so sichergestellt werden, dass die Monomerflüssigkeit nicht ohne Einwirkung des Unterdrucks ungewollt in die Kartusche fließen kann und dort ungewollt aushärtet. Damit kann verhindert werden, dass die Monomerflüssigkeit beim Öffnen des separaten oder integrierten Behälters (beispielsweise der Monomer-Glasampulle) bereits durch die Flüssigkeitsleitung in den Innenraum der Kartusche gelangt. Diese Schlaufe, der beispielsweise durch eine umgekehrt U-förmige Schlaufe der Fluidverbindung aufgebaut werden kann, erreicht, dass vor Bewegung des Kolbens der Pumpe die Monomerflüssigkeit innerhalb der Vakuummischvorrichtung bis zur Höhe des Scheitelpunkts in der Fluidverbindung verbleibt, wodurch ein vorzeitiger Eintritt der Monomerflüssigkeit zum Zementpulver verhindert wird. Insbesondere bei hochviskosen Zementen kann ein vorzeitiger Kontakt von schon geringen Volumina der Monomerflüssigkeit mit dem Zementpulver zur Verklebung der Fluidverbindung oder einer als Düse ausgebildeten Fluidverbindung, wie in US 8 662 736 B2 beschrieben, führen. Die Fluidverbindung kann transparent oder transluzent sein, damit der Anwender den Monomertransfer visuell kontrollieren kann. Hierzu kann insbesondere ein Sichtfenster in der Vakuummischvorrichtung vorgesehen sein, durch das die Schlaufe mit dem höchsten Scheitelpunkt zu erkennen ist.

Mit einer Weiterbildung der vorliegenden Erfindung wird ferner vorgeschlagen, dass durch die Bewegung des Kolbens in der Pumpe im Pumpraum ein Unterdruck zu erzeugen ist, wobei mit dem Unterdruck ein Gas durch die Verbindungsleitung aus dem Innenraum der zumindest einen Kartusche evakuierbar ist.

Hiermit wird ein besonders einfacher und unanfälliger Aufbau bereitgestellt.

Erfindungsgemäß kann auch vorgesehen sein, dass die Pumpe aufgebaut ist mit einem Hohlzylinder, wobei der Hohlzylinder mit dem Innenraum der Kartusche verbunden oder verbindbar ist, einem gasdichten Verschluss an einem Hohlzylinderende, dem Kolben, der im Hohlzylinder gasdicht, axial beweglich angeordnet ist, wobei der Kolben in der Pumpe mit dem manuell bedienbaren Bedienelement bewegbar ist, wobei bei einem Bewegen des Kolbens mit dem manuell bedienbaren Bedienelement der Kolben axial entgegengesetzt zum Verschluss bewegbar ist und dadurch Gas aus dem Innenraum der Kartusche evakuiert, wobei das Bedienelement mit der Öffnungseinrichtung in Wirkverbindung steht und wobei das Bedienelement mit der Mischeinrichtung im Innenraum der Kartusche derart verbunden ist, dass bei einem Bedienen des Bedienelements die Mischeinrichtung im Innenraum der Kartusche bewegbar ist.

Dieser Aufbau ist besonders einfach und die wesentlichen Teile hierfür können aus Kunststoff durch Spritzgießen gefertigt werden.

Mit einer besonders bevorzugten Ausführung der vorliegenden Erfindung kann vorgesehen sein, dass die Pumpe, die Öffnungseinrichtung und die Mischeinrichtung über die Bewegung des Bedienelements antreibbar sind, wobei bevorzugt die Bewegung des Bedienelements durch manuelle Krafteinwirkung erfolgt.

Hierdurch wird erreicht, dass die Vakuummischvorrichtung keinerlei Energiespeicher und keine elektrischen oder elektronischen Antriebe benötigt. Dies ist deswegen erstrebenswert, da die Vakuummischvorrichtung zum einmaligen Gebrauch bestimmt ist und auf diese Weise leichter zu recyceln ist. Zudem kann so erreicht werden, dass die Vakuummischvorrichtung grundsätzlich anwendbar ist und keine Anschlüsse, wie Kabel oder Druckgasschläuche benötigt, um eingesetzt werden zu können.

Die der vorliegenden Erfindung zugrundeliegenden Aufgaben werden auch gelöst durch ein Verfahren zur Vermischung von Polymethylmethacrylat-Knochenzement in einem Innenraum einer Kartusche einer Vakuummischvorrichtung, insbesondere einer solchen, oben beschriebenen Vakuummischvorrichtung, bei dem ein Bedienelement bedient wird und dadurch ein integrierter Behälter der Vakuummischvorrichtung oder ein separater Behälter, der in einer Aufnahme der Vakuummischvorrichtung angeordnet ist, geöffnet wird, wobei anschließend eine in dem integrierten Behälter oder dem separaten Behälter enthaltene Monomerflüssigkeit als erste Komponente des Knochenzements ausläuft,
durch eine anschließende weitere Bedienung des Bedienelements eine Bewegung eines Kolbens einer Pumpe der Vakuummischvorrichtung angetrieben wird, wobei durch die Bewegung des Kolbens ein Unterdruck in einem Pumpraum der Pumpe erzeugt wird, wobei mit der derart angetriebenen Pumpe der Innenraum der Kartusche evakuiert wird und mit dem Unterdruck im Innenraum der Kartusche die Monomerflüssigkeit in den Innenraum der Kartusche geleitet wird, wobei sich in dem Innenraum der Kartusche bereits ein Knochenzementpulver als zweite Komponente des Knochenzements befindet, und
durch die Bedienung des Bedienelements eine Mischeinrichtung im Innenraum der Kartusche bewegt wird und durch die Bewegung der Mischeinrichtung ein Knochenzementteig im Innenraum der Kartusche aus dem Zementpulver und der Monomerflüssigkeit gemischt wird.

Dabei kann vorgesehen sein, dass das Volumen eines Pumpraums der Pumpe durch die manuelle Bewegung des Kolbens vergrößert wird und durch den dadurch entstehenden Unterdruck der Innenraum der Kartusche evakuiert wird.

Hiermit kann die Pumpe auf einfache Weise realisiert werden.

Ferner kann erfindungsgemäß vorgesehen sein, dass ein Zementpulver in dem Innenraum der Kartusche enthalten ist und ein Gas aus dem Innenraum der Kartusche mit der Pumpe evakuiert wird, eine Monomerflüssigkeit in den Innenraum der Kartusche eingeleitet wird und die Monomerflüssigkeit mit dem Zementpulver in dem evakuierten Innenraum der Kartusche durch eine Bewegung der Mischeinrichtung gemischt wird.

Durch die genannte Kombination und Wechselwirkung der Verfahrensschritte kann ein besonders einfacher und sicher zu realisierender Aufbau erreicht werden.

Des Weiteren kann vorgesehen sein, dass der Kolben der Pumpe mit dem Bedienelement bewegt wird, wodurch ein Unterdruck gegenüber der umgebenden Atmosphäre in der Pumpe erzeugt wird, dabei durch eine Verbindungsleitung Gas aus dem Innenraum der Kartusche in den Pumpraum der Pumpe gesaugt wird, anschließend durch Bedienen desselben Bedienelements die Vakuummischvorrichtung im Innenraum der Kartusche bewegt wird und dabei das Zementpulver mit der Monomerflüssigkeit vermischt wird, anschließend die Kartusche mit dem gemischten Zementteig entnommen wird und durch axiale Bewegung eines Austragskolbens der Zementteig aus der Kartusche heraus gepresst wird.

Hiermit wird das Verfahren dahingehend komplettiert, dass am Ende eine Zementkartusche mit einem unter Vakuum gemischten Knochenzementteig bereitgestellt wird, der unmittelbar angewendet werden kann.

Schließlich kann auch vorgesehen sein, dass das Zementpulver in der Kartusche angeordnet ist, die Monomerflüssigkeit in einer von der Kartusche separaten Aufnahme angeordnet ist, wobei die Monomerflüssigkeit in einem integrierten Behälter oder in einem separaten Behälter, vorzugsweise in einer Glasampulle in der Aufnahme, enthalten ist, der integrierte Behälter oder der separate Behälter durch Bedienen des Bedienelements und einer daraus resultierenden Bewegung der Öffnungseinrichtung geöffnet wird, bevor der Kolben durch ein weiteres Bedienen des Bedienelements angetrieben wird, danach der Kolben axial in einem Hohlzylinder bewegt wird, wodurch ein Unterdruck gegenüber der umgebenden Atmosphäre erzeugt wird, dabei durch die Verbindungsleitung Gas aus dem Innenraum der Kartusche in den Hohlzylinder gesaugt wird und durch den in dem Innenraum der Kartusche gebildeten Unterdruck Monomerflüssigkeit in die Kartusche gesaugt wird.

Dadurch wird das Verfahren weiter komplettiert.

Erfindungsgemäße Verfahren können auch durch die bestimmungsgemäße Anwendung oder Verwendung von Bauteilen erfindungsgemäßer Vakuummischvorrichtungen gekennzeichnet sein.

Der Erfindung liegt die überraschende Erkenntnis zugrunde, dass es mit einem einzigen Bedienelement gelingt, sowohl die Pumpe und Mischeinrichtung zu betreiben beziehungsweise anzutreiben, als auch die Öffnungseinrichtung zu bedienen. Dies hat den Vorteil, dass keine komplizierten Handlungsanweisungen an das bedienende Personal notwendig sind. Durch Bedienen des einzigen Bedienelements können alle Abläufe gesteuert und angetrieben werden. Die Vakuummischvorrichtung wird so maximal vereinfacht. Gleichzeitig sind keine Energiespeicher zum Antrieb notwendig und es bedarf keiner elektrischen beziehungsweise elektronischen Steuerung, um die Pumpe, die Mischeinrichtung und die Öffnungseinrichtung anzutreiben und zu steuern.

Gleichzeitig gelingt es mit Hilfe der manuell anzutreibenden Pumpe, eine von inneren und äußeren Energiequellen und anderen Versorgungsleitungen unabhängige Vakuummischvorrichtung bereitzustellen. Die erfindungsgemäße Vakuummischvorrichtung kann kompakt, leicht und platzsparend aufgebaut werden. Die Pumpe, die Öffnungseinrichtung und die gesamte Vakuummischvorrichtung können mit einfachsten Mitteln aufgebaut werden, so dass die gesamte Vakuummischvorrichtung als Einmalsystem verwendet werden kann. Die Pumpe kann ferner und erfindungsgemäß bevorzugt auch dazu eingesetzt werden, um eine Monomerflüssigkeit in das Zementpulver zu überführen. Die beiden Komponenten des PMMA-Knochenzements können dann im Vakuum beziehungsweise in dem Unterdruck gemischt werden.

In Zementiersystemen nach der vorliegenden Erfindung ist eine Vorrichtung zur Erzeugung eines Vakuums beziehungsweise zur Erzeugung eines Unterdrucks enthalten, die zur temporären Erzeugung eines Unterdrucks vor und während der Vermischung einer pulverförmigen Komponente mit einer flüssigen Monomerkomponente des Polymethylmethacrylat-Knochenzements geeignet ist.

Die der Erfindung zugrundeliegende Idee basiert auf der Erkenntnis, dass nur eine relativ geringe Energiemenge und damit ein geringer manueller Kraftaufwand notwendig ist, um den Behälter für die Monomerflüssigkeit zu öffnen, um das Vakuum beziehungsweise den Unterdruck in einer Kartusche zu erzeugen, der notwendig ist, um die Ausgangskomponenten eines Knochenzements unter dem Unterdruck oder dem Vakuum zu mischen, und um die Mischeinrichtung zum Durchmischen des Knochenzementteigs im Innenraum der Kartusche zu bewegen. Die Energiemenge, um die Monomerflüssigkeit in das Zementpulver zu überführen, ist ebenfalls gering. Diese geringe Energiemenge kann ohne weiteres durch Bedienen eines Hebels als Bedienelement aufgebracht werden. Hierdurch ist die Vakuummischvorrichtung einfach in der Handhabung und leicht zu bedienen sowie unabhängig von inneren und äußeren Energiespeichern. Durch einen geeigneten Aufbau lässt sich auch die Reihenfolge der Abläufe steuern, nämlich dass zuerst der Monomerbehälter geöffnet wird und erst anschließend die Pumpe und die Mischeinrichtung angetrieben werden.

Die Idee der Erfindung beruht auch darauf, dass durch manuelle Betätigung eines Bedienelements mit einem damit verbundenen Kolben in einem Hohlzylinder der Pumpe ein Unterdruck in dem Hohlzylinder der Pumpe erzeugt wird, wobei sich der Unterdruck über ein Leitungsmittel in die Kartusche ausbreitet, und die Monomerflüssigkeit aus einem geöffneten Monomerflüssigkeitsbehälter in die Kartusche gesaugt wird, in dem sich Zementpulver befindet. Danach erfolgt eine manuelle Vermischung der Zementkomponenten mit Hilfe einer Mischeinrichtung, die über dasselbe Bedienelement zeitgleich anzutreiben ist.

Beispielsweise kann die Erfindung mit dem folgenden Verfahren umgesetzt werden, bei dem durch Aktivierung mit einem manuell zu bedienenden Hebel oder allgemeiner einem manuell zu bedienenden Bedienelement die folgenden Funktionen in der Mischvorrichtung ablaufen:
1. Schritt: Betätigung des Handhebels beziehungsweise des Bedienelements und Brechen der Ampulle beziehungsweise Ampullen, Auslaufen der Monomerflüssigkeit innerhalb von 1 bis 2 Sekunden in ein Reservoir, das mit einem Leitungsmittel und einer Düse verbunden ist, wobei die Düse mit der Öffnung in den Innenraum der Kartusche zeigt, beim Anschlagpunkt des Hebels Einrasten einer elastischen Stange in ein Gegenrastmittel des Hebels, wobei die Stange in einen ersten Teil und einen zweiten Teil gegabelt ist;
2. Schritt: Rückwärtsbewegung des Handhebels beziehungsweise des Bedienelements in die Ausgangsposition, wobei durch den ersten Teil der elastischen Stange ein Kolben in einem Hohlzylinder axial bewegt wird und ein Unterdrück hinter dem Kolben im Hohlzylinder entsteht, Weiterleitung des Unterdrucks über ein Rückschlagventil, ein Leitungsmittel zum Vakuumanschluss in den Innenraum der Kartusche, Ausbildung eines Unterdrucks im Innenraum der Kartusche, Ansaugen der Monomerflüssigkeit aus dem Reservoir in die Kartusche, Bewegung des zweiten Teils der elastischen Stange, die mit einer ersten Hülse drehbar verbunden ist, die mindestens eine äußerte Nocke besitzt, die in einem Steilgewinde einer zweiten Hülse beweglich angeordnet ist, dass ein elastischer Rührstab (als Mischwelle) mit der ersten Hülse fest verbunden ist, so dass bei axialer Bewegung der ersten Hülse durch die (beziehungsweise in der) zweiten Hülse die erste Hülse durch Eingriff der Nocke in das Steilgewinde gedreht wird, wodurch der Rührstab und rotiert und sich axial in der Kartusche bewegt;
3. Schritt: Weitere Betätigung des Handhebels beziehungsweise des Bedienelements, Bewegung der ersten Hülse in der zweiten Hülse, axiale Bewegung des Rührstabs in der Kartusche unter Drehung um die Längsachse;
4. Schritt: Wiederholung des 2. und 3. Schritts bis der Zementteig homogen gemischt ist;
5. Schritt: Ablösen der Kartusche beziehungsweise des Kartuschensystems durch Herausschrauben und Herausziehen des Mischstabs (der Mischwelle) mit dem Mischelement, unter Umklappen der Mischelemente (der Mischflügel der Mischeinrichtung).

Der wesentliche Vorteil der Erfindung ist, dass ein Prepack-Mischsystem vorgeschlagen wird, das in einfachster Weise, ohne spezielle Schulungsmaßnahmen, durch den medizinischen Anwender durch einfach manuelle Betätigung genutzt kann, um innerhalb von wenigen Sekunden (etwa innerhalb von 40 Sekunden) einen Polymethylmethacrylat-Knochenzementteig herzustellen. Vorteilhaft ist weiterhin, dass auf Grund der maximal vereinfachten Bedienung Anwenderfehler minimiert werden und dadurch die Patientensicherheit verbessert wird.

Die erfindungsgemäße Vakuummischvorrichtung kann im Wesentlichen kostengünstig mit einfachen, durch Kunststoffspritzgießen herzustellenden Kunststoffteilen realisiert werden. Der besondere Vorteil der erfindungsgemäßen Vorrichtung besteht darin, dass die Vorrichtung ohne äußere Hilfsmittel, wie durch Druckluft angetriebene Vakuumpumpen und Vakuumschläuche, und ohne Energiequellen, wie Druckluft oder Batterien, betrieben werden kann. Die erfindungsgemäße Vakuummischvorrichtung ist autonom verwendbar und kann selbst unter einfachsten beziehungsweise schwierigsten Operationsbedingungen verwendet werden. Mit der erfindungsgemäßen Vakuummischvorrichtung wird ein geschlossenes Full-Prepack-Vakuumzementiersystem für preissensitive Märkte zur Verfügung gestellt.

Im Folgenden werden weitere Ausführungsbeispiele der Erfindung anhand von acht schematisch dargestellten Figuren erläutert, ohne jedoch dabei die Erfindung zu beschränken. Dabei zeigt:
- Figur 1:: eine schematische perspektivische Ansicht einer erfindungsgemäßen Vakuummischvorrichtung;
- Figur 2:: eine schematische perspektivische Ansicht der Vakuummischvorrichtung nach Figur 1 mit geöffnetem Gehäuse;
- Figur 3:: eine schematische perspektivische Querschnittansicht der Vakuummischvorrichtung nach den Figuren 1 und 2 im Ausgangszustand;
- Figur 4:: eine schematische Querschnittansicht der Vakuummischvorrichtung nach den Figuren 1 bis 3 im Ausgangszustand;
- Figur 5:: eine schematische Querschnittansicht der Vakuummischvorrichtung nach den Figuren 1 bis 4 mit einer Schnittebene senkrecht zu dem Schnitt der Figuren 3 und 4;
- Figur 6:: eine schematische Querschnittansicht der Vakuummischvorrichtung nach den Figuren 1 bis 5 während der Bedienung mit aufgebrochener Glasampulle;
- Figur 7:: eine schematische Querschnittansicht der Vakuummischvorrichtung nach den Figuren 1 bis 6 während der Bedienung mit eingerastetem Kabel oder eingerasteter Stange; und
- Figur 8:: eine schematische Querschnittansicht der Vakuummischvorrichtung nach den Figuren 1 bis 7 während der Bedienung beim Pumpen und Mischen.

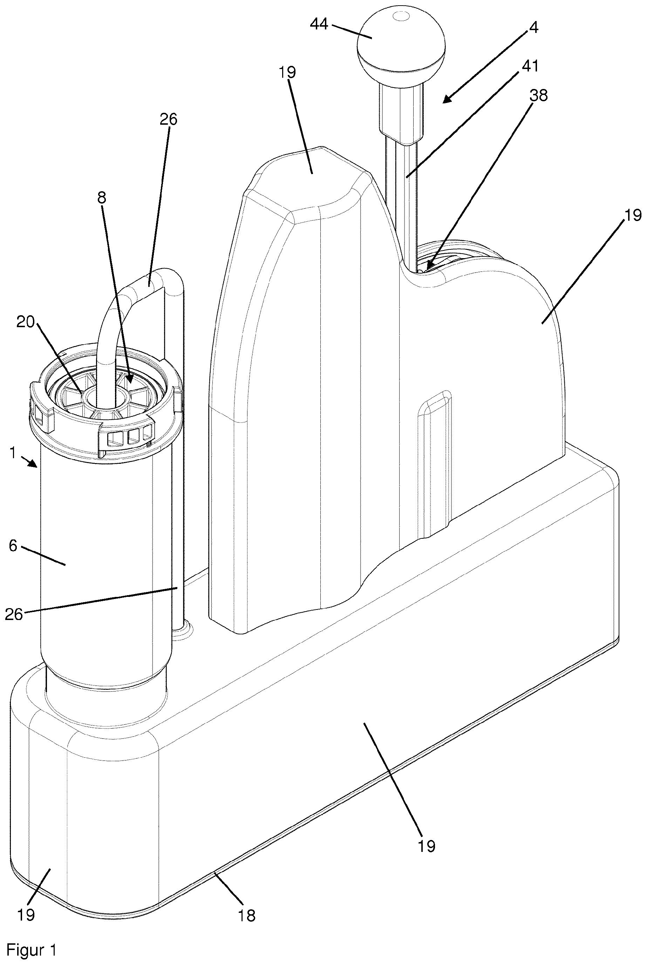

Die Figuren 1 bis 8 zeigen verschiedene Ansichten einer erfindungsgemäßen Vakuummischvorrichtung vor und während des Betriebs. Die Vakuummischvorrichtung besteht im Wesentlichen aus fünf Teilen, nämlich einem Kartuschensystem 1, einem Flüssigkeitsbehälter 2, einer manuell anzutreibenden Pumpe 3, einem Bedienelement 4 und einer Öffnungseinrichtung 5.

Zentraler Bestandteil des Kartuschensystems 1 ist eine Kartusche 6 mit einem zylindrischen Innenraum, die an ihrer Oberseite durch einen zweiteiligen Austragskolben 8 verschlossen ist, der im zylindrischen Innenraum der Kartusche 6 in Längsrichtung beweglich angeordnet ist. Die Kartusche 6 hat also einen zylindrischen Innenraum mit kreisförmiger Grundfläche. Die Kartusche 6 enthält ein Zementpulver 9 als Ausgangskomponente für einen Knochenzement.

Im Innenraum der Kartusche 6 ist ferner eine Mischeinrichtung 10 mit zwei oder mehr Mischflügeln 10 angeordnet, wobei die Mischeinrichtung 10 drehbar und in Längsrichtung verschiebbar im Innenraum der Kartusche 6 gelagert ist und die an einer Mischwelle 12 oder an einem Kabel 12 befestigt ist, die oder das drehbar und in Längsrichtung verschiebbar durch eine Durchführung in der Unterseite der Kartusche 6 in den Innenraum der Kartusche 6 geführt ist. Die Durchführung ist hierzu druckdicht und gasdicht ausgeführt. Die Mischwelle 12 kann auch als flexible Stange 12 ausgeführt werden.

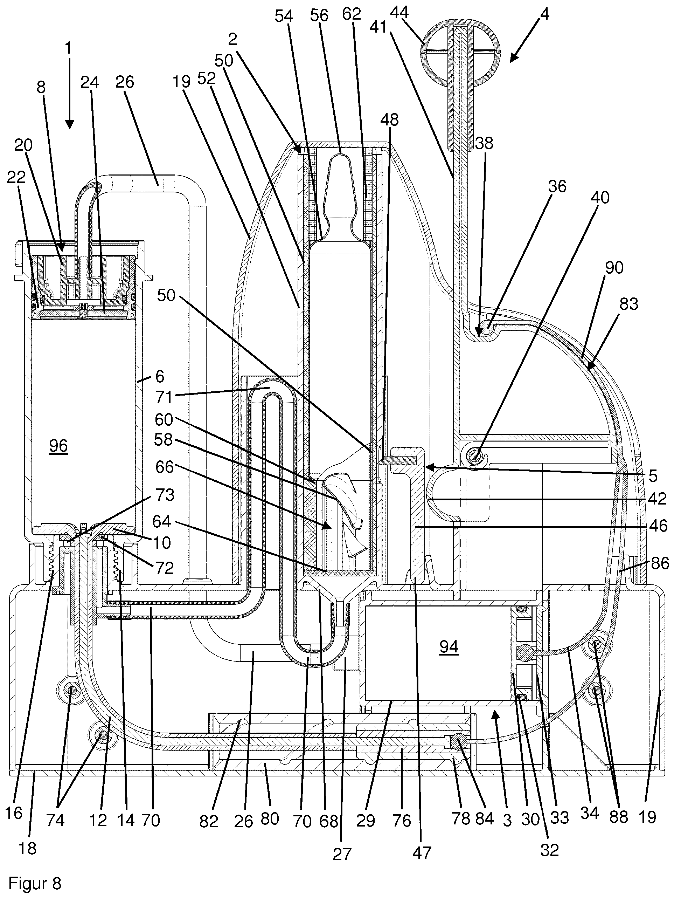

Das Kartuschensystem 1 ist mit dem Flüssigkeitsbehälter 2 und der Pumpe 3 über ein Fußteil 18 und ein Gehäuse 19 verbunden. Der Flüssigkeitsbehälter 2, die Pumpe 3, ein Teil des Bedienelements 4 und die Öffnungseinrichtung 5 sind von dem Gehäuse 19 umschlossen, wobei ein Teil des Bedienelements 4 aus dem Gehäuse 19 herausragt, während das Kartuschensystem 1 auf das Gehäuse 19 aufgeschraubt ist. Die Kartusche 6 endet an ihrer Unterseite in einem Stutzen mit einem Innengewinde 14, das auf ein Außengewinde 16 an einem Stutzen des Gehäuses 19 geschraubt ist. Das Fußteil 18 bildet dabei den Standfuß 18 der kompakten Vakuummischvorrichtung. Die Kartusche 6 ist dadurch von dem Gehäuse 19 und damit dem Rest der Vakuummischvorrichtung lösbar. Wenn der Knochenzement 96 (siehe Figur 8) mit der Vakuummischvorrichtung im Innenraum der Kartusche 6 fertig gemischt ist, kann also die Kartusche 6 vom Gehäuse 19 abgeschraubt werden und in das Innengewinde 14 kann ein Austragsrohr (nicht gezeigt) eingeschraubt werden, durch das der fertige Knochenzementteig 96 (siehe Figur 8) durch Vortreiben des Austragskolbens 8 in Richtung des Innengewindes 14 ausgetrieben werden kann. Im Austragsrohr kann ein statischer Mischer vorgesehen sein, der eine zusätzliche Durchmischung des Knochenzementteigs 96 bewirkt.

Der zweiteilige Austragskolben 8 hat einen Dichtungskolben 20 und einen Sterilisationskolben 22. Der Sterilisationskolben 22 weist eine Membran oder Porenscheibe 24 auf, die für ein sterilisierendes Gas durchlässig ist aber für das Zementpulver 9 nicht durchlässig ist. Der Sterilisationskolben 22 wird nach dem Einfüllen des Zementpulvers 9 in die Kartusche 6 eingesetzt und schließt den Innenraum der Kartusche 6 nach außen ab. Anschließend kann der Inhalt der Kartusche 6 durch die gasdurchlässige Membran oder Porenscheibe 24 mit Ethylendioxid sterilisiert werden.

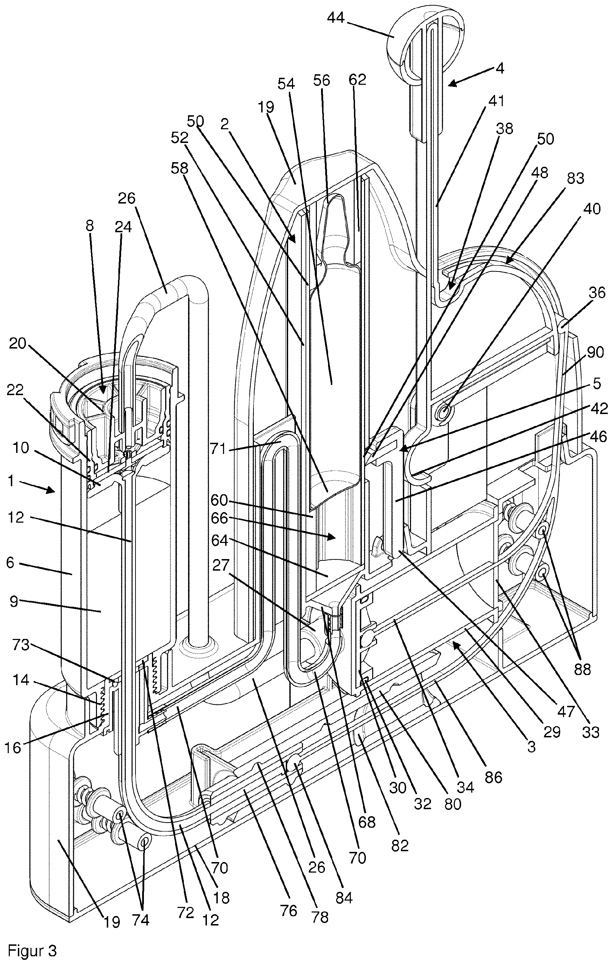

Der Dichtungskolben 20 kann in den Sterilisationskolben 22 gedrückt und mit diesem gas- und druckdicht verbunden werden. Die aneinander befestigten Kolbenteile 20, 22 bilden dann zusammen den Austragskolben 8, mit dem der Inhalt der Kartusche 6 durch die Öffnung im Stutzen mit dem Innengewinde 14 herausgepresst werden kann. Zunächst ist der Sterilisationskolben 22 auf der mit der Öffnung in dem Stutzen mit dem Innengewinde 14 gegenüberliegenden Seite (in den Figuren 3, 4 und 6 bis 8 oben) arretiert, wobei die Arretierung lösbar ist. Durch die Arretierung wird verhindert, dass sich der Sterilisationskolben 22 während der Sterilisation des Innenraums der Kartusche 6 sowie des Zementpulvers 9 ungewollt bewegt.

Über die Mischwelle 12 beziehungsweise das Kabel 12 können die Mischflügel 10 im Inneren der Kartusche 6, also im Innenraum der Kartusche 6 gedreht werden und in Längsrichtung der Kartusche 6 bewegt werden.

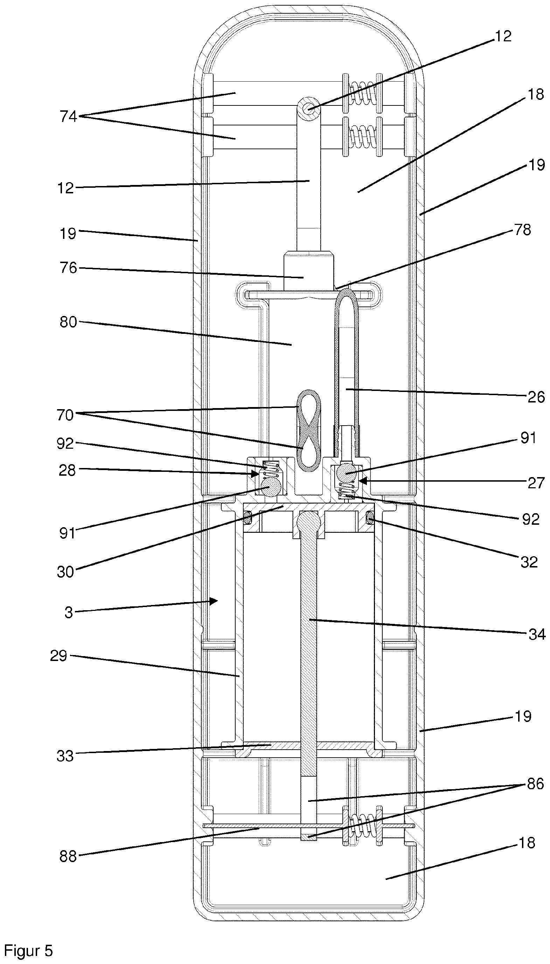

In dem Dichtungskolben 20 ist eine Durchführung vorgesehen, die an eine Verbindungsleitung 26 in Form einer flexiblen Vakuumleitung 26 angeschlossen ist. Der Dichtungskolben 20 schließt ansonsten druckdicht mit der Kartusche 6. Die Vakuumleitung 26 mündet über ein erstes Rückschlagventil 27 in die Pumpe 3, so dass nur eine Strömung in Richtung in die Pumpe 3 hinein möglich ist. Die Pumpe 3 ist zudem über ein zweites Rückschlagventil 28 mit der Umgebung verbunden, so dass ein in der Pumpe 3 entstehender Überdruck über das zweite Rückschlagventil 28 abgeblasen werden kann. Details zu den Rückschlagventilen 27, 28 sind in Figur 5 dargestellt und in der Beschreibung weiter unten ausgeführt. An dem zweiten Rückschlagventil 28 kann optional ein Filter (nicht gezeigt) vorgesehen sein, mit dem Methacrylatdämpfe oder andere störende chemische Substanzen aus dem ausströmenden Gas herausgefiltert werden.

Die Pumpe 3 weist einen stabilen Hohlzylinder 29 auf. Der Hohlzylinder 29 ist über einen Kolben 30 druckdicht in zwei Teile getrennt. Hierzu weist der Kolben 30 eine umlaufende Dichtung 32 auf, die mit der Innenwand des Hohlzylinders 29 abschließt. Der Kolben 30 ist mit einem Kabel 34 oder einer flexiblen Stange 34 aus einem stabilen elastischen Kunststoff oder aus einem Metall wie Stahl verbunden, das durch eine Durchführung in einem rückseitigen Verschluss 33 führt. Auf der Vorderseite ist die Pumpe 3 durch einen vorderseitigen Verschluss verschlossen, in dem sich die Anschlüsse für die Rückschlagventile 27, 28 befinden. Der Verschluss 33 ist auf der den Rückschlagventilen 27, 28 gegenüberliegenden Seiten des Hohlzylinders 29 angeschlossen. Die Vakuumleitung 26 ist bis zu der Pumpe 3 geführt, so dass die Durchführung im Dichtungskolben 20 über die Vakuumleitung 26 druckdicht mit der Pumpe 3 genauer mit einem Pumpraum 94 (siehe Figur 8) der Pumpe 3 verbunden ist. Der Pumpraum 94 ist durch die Innenwände des Hohlzylinders 29, durch den vorderseitigen Verschluss und durch den Kolben 30 begrenzt.

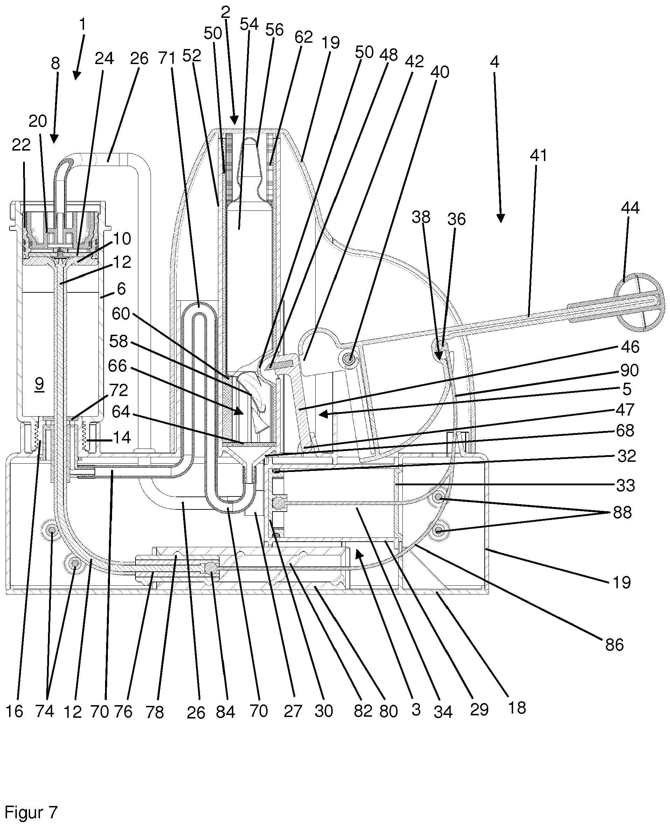

Das Kabel 34 ist mit einem Rastmittel 36 verbunden, das mit einem Gegenrastmittel 38 rasten kann (siehe Figuren 7 und 8). Das Gegenrastmittel 38 ist Teil des Bedienelements 4, das als ein um eine Achse 40 drehbarer beziehungsweise schwenkbarer Hebel 4 aufgebaut ist. Der Hebel 4 umfasst zwei Hebelarme 41, 42, die sich von der Achse 40 aus in unterschiedliche Richtungen erstrecken. Das eigentliche Bedienteil des Hebels 4 bildet der erste Hebelarm 41, der in einem Griff 44 endet, der von außen manuell bedienbar ist. Der erste Hebelarm 41 ragt also aus dem Gehäuse 19 heraus und der Griff 44 ist außerhalb des Gehäuses 19 angeordnet und kann vom Anwender der Vakuummischvorrichtung manuell bedient werden. Dabei kann über den ersten Hebelarm 41 eine beträchtliche Kraft ins Innere der Vakuummischvorrichtung übertragen werden, die zum Antreiben der Öffnungseinrichtung 5, der Pumpe 3 und der Mischeinrichtung 10 ausreicht und erfindungsgemäß angewendet wird.

Der zweite Hebelarm 42 drückt beim Schwenken des Hebels 4 beziehungsweise beim nach unten drücken des Hebels 4 auf die Öffnungseinrichtung 5 und treibt diese an. Dazu ist die Öffnungseinrichtung 5 mit einem Hebel 46 aufgebaut, der um eine Achse 47 drehbar beziehungsweise schwenkbar gelagert ist. An dem der Achse 47 gegenüberliegenden Ende des Hebels 46 ist ein Einsatz mit einer Kante 48 vorgesehen, die an der Aufnahme 2 beziehungsweise dem Flüssigkeitsbehälter 2 anliegt.

Der Flüssigkeitsbehälter 2 beziehungsweise die Aufnahme 2 umfasst einen inneren elastischen Einsatz 50, der beispielsweise aus einem Gummi bestehen kann, und einen starren Hohlzylinder 52 aus einem Kunststoff wie Plastik. In der Aufnahme 2 beziehungsweise dem Flüssigkeitsbehälter 2 ist eine Glasampulle 54 enthaltend eine Monomerflüssigkeit eingesteckt. Die Monomerflüssigkeit bildet mit dem Zementpulver 9 aus der Kartusche 6 einen Knochenzementteig 96, wenn sie miteinander durchmischt werden. Die Innenwandungen des elastischen Einsatzes 50 liegen an der Glasampulle 54 an. Die Glasampulle 54 weist einen Ampullenkopf 56 und einen dem Ampullenkopf 56 gegenüberliegenden Ampullenboden 58 auf. Die Glasampulle 54 sitzt mit dem Ampullenboden 58 auf einer Auflage 60 auf, die als Absatz 60 des elastischen Einsatzes 50 ausgeformt ist. Auf der Oberseite drückt ein Hohlzylinder 62 aus einem gasdurchlässigen Schaumstoff, der an der Innenseite des Gehäuses 19 befestigt ist, die Glasampulle 54 auf die Auflage 60. Zwischen dem Hohlzylinder 52 und dem Gehäuse 19 sind Öffnungen vorgesehen, durch die Luft oder Gas aus der Umgebung der Aufnahme 2 und der Vakuummischvorrichtung in die Aufnahme 2 nachströmen kann. Dadurch kann die Monomerflüssigkeit leichter aus der Aufnahme 2 abfließen. Die Öffnungen zwischen dem Hohlzylinder 52 und dem Gehäuse 19 sind derart gestaltet, dass sie in der Zylindermantelwand des Hohlzylinders 52 angrenzend an die obere Grundfläche des Hohlzylinders 52 angeordnet sind. Der Hohlzylinder 52 liegt also nur bereichsweise an dem Gehäuse 19 an. Dazwischen kann Luft ins Innere der Aufnahme 2 nachströmen.

Im Bereich des Ampullenbodens 58 beziehungsweise der Auflage 60 weist der Hohlzylinder 52 eine Ausnehmung auf, innerhalb der die Kante 48 an dem elastischen Einsatz 50 anliegt, so dass der Ampullenboden 58 mit der Öffnungseinrichtung 5 abgebrochen und damit die Glasampulle 54 geöffnet werden kann. Der Ampullenkopf 56 der Glasampulle 54 wird üblicherweise zum Öffnen der Glasampulle 54 abgebrochen. Da die Glasampulle 54 am Hals dünn ausgeführt ist, führt dies jedoch dazu, dass die Monomerflüssigkeit aus der Glasampulle 54 nur langsam auslaufen kann und daher der Anwender warten muss, bis er die nächsten Schritte zum Bedienen der Vakuummischvorrichtung durchführen kann. Dies ist bei dem weitgehend automatisierten Verfahren, das durch Bedienen des Hebels 4 beziehungsweise des Bedienelements 4 angetrieben wird nicht geeignet, da so nicht sichergestellt werden könnte, dass die Monomerflüssigkeit aus der Glasampulle 54 schon zur Verfügung steht, wenn die Pumpe 3 über das Bedienelement 4 angetrieben wird.

Die Glasampulle 54 steckt in dem Einsatz 50 aus dem verformbaren Material. Der Einsatz 50 bildet zusammen mit dem Hohlzylinder 52 die wesentlichen Teile der Aufnahme 2 für die Glasampulle 54. Die Glasampulle 54 kann aufgrund des Absatzes 60 nur bis zum Ampullenboden 58 in den Einsatz 50 des Flüssigkeitsbehälters 2 eingeschoben werden.

Der Flüssigkeitsbehälter 2 weist eine seitliche Öffnung auf, in der der Einsatz 50 eine verformbare Seitenwand bildet. An dieser Stelle kann die Glasampulle 54 geöffnet beziehungsweise aufgebrochen werden, indem ein Druck durch die verformbare Seitenwand 50 auf die Glasampulle 54 knapp oberhalb des Ampullenbodens 58 wirkt. Wenn der Ampullenboden 58 der Glasampulle 54 abgebrochen beziehungsweise die Glasampulle 54 geöffnet wird, kann die Monomerflüssigkeit aus der geöffneten Glasampulle 54 im vollen Querschnitt ausfließen, so dass die Monomerflüssigkeit schnell im vollen Umfang zur Weiterverarbeitung innerhalb der Vakuummischvorrichtung zur Verfügung steht.

Um die verformbare Seitenwand 50 zu verformen und dadurch die Glasampulle 54 aufzubrechen, wird der Hebel 42 verwendet, der über den Hebel 4 bedienbar ist und der um die Achse 40 gedreht werden kann. Der Hebel 4 ist schwenkbar beziehungsweise drehbar um die Achse 40 gegen das Gehäuse 19 gelagert. Die Achse 40 teilt den Hebel 4 in einen langen Hebelarm 41, an dem der Griff 44 befestigt ist und einen kurzen Hebelarm 42 der innerhalb des Gehäuses 19 angeordnet ist. Zu Beginn kann der lange Hebelarm 41 nur von dem Flüssigkeitsbehälter 2 weg bewegt werden und nicht auf diesen zu, da der lange Hebelarm 41 oben an der Öffnung des Gehäuses 19 anliegt und so eine weitere Bewegung in diese Richtung unterbindet.

Der kurze Hebelarm 42 des Hebels 4 liegt auf seiner dem Flüssigkeitsbehälter 2 zugewandten Seite an dem Hebel 46 der Öffnungseinrichtung 5 an, der über ein Gelenk 47 beziehungsweise die Achse 47 drehbar um die Achse 47 mit dem Fußteil 18 beziehungsweise dem Gehäuse 19 der Vakuummischvorrichtung verbunden ist. Dieser Hebel 46 der Öffnungseinrichtung 5 ist innerhalb des Gehäuses 19 angeordnet. Das freie Hebelende des Hebels 46 im Gehäuse 19 kann mit dem kurzen Hebelarm 42 bewegt werden. An der Spitze des freien Hebelendes ist die Kante 48 befestigt, die an der verformbaren Seitenwand 50 anliegt. Die Achse 47 des Hebels 46 ist dabei so angeordnet, dass sich das freie Hebelende und damit die Kante 48 in Richtung der verformbaren Seitenwand 50 und in Richtung des Fußteils 18 bewegt. Dadurch wird erreicht, dass die Kraft, die von der Kante 48 durch die verformbare Seitenwand 50 auf die Glasampulle 54 ausgeübt werden kann, die Glasampulle 54 auch leicht in Richtung des Absatzes 60 presst und somit die Glasampulle 50 in die Aufnahme 2 hineindrückt.

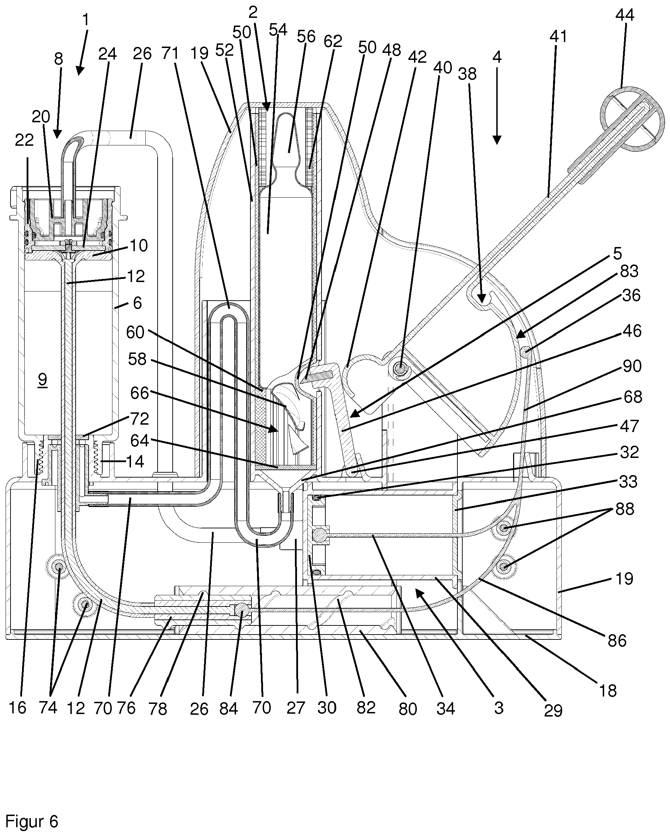

Unterhalb des Absatzes 60 ist ein Sieb 64 und/oder ein Filter 64 angeordnet, mit dem oder mit denen Glassplitter der geöffneten beziehungsweise aufgebrochenen Glasampulle 54 zurückgehalten werden. Der Abstand zwischen dem Absatz 60 und dem Sieb 64 und/oder Filter 64 ist größer als der Außendurchmesser der Glasampulle 54, so dass der abfallende Ampullenboden 58 sich in dem Zwischenraum drehen kann und den Ausfluss der Monomerflüssigkeit aus der geöffneten Glasampulle 54 nicht behindert (siehe Figuren 6 bis 8). Die Monomerflüssigkeit verbleibt dann zumindest teilweise in einem Speichervolumen 66, das unterhalb der Glasampulle 54 angeordnet ist und in dem das Sieb 64 und/oder der Filter 64 angeordnet ist. Unterhalb des Siebs 64 und/oder Filters 64 ist ein Trichter 68 angeordnet, der in eine Fluidverbindung 70 beziehungsweise Flüssigkeitsleitung 70 mündet.

Die Vorderseite der Kartusche 6 (in den Figuren 1 bis 4 und 6 bis 8 unten) ist durch das Fußteil 18 und das Gehäuse 19 über die Fluidverbindung 70 dicht mit dem Speichervolumen 66 beziehungsweise dem Trichter 68 des Flüssigkeitsbehälters 2 verbunden. In der Fluidverbindung 70 ist eine Schlaufe 71 vorgesehen, mit der verhindert wird, dass eine in dem Flüssigkeitsbehälter 2 beziehungsweise dem Speichervolumen 66 enthaltene Monomerflüssigkeit (nicht gezeigt) ungewollt bis in den Innenraum der Kartusche 6 vordringen kann.

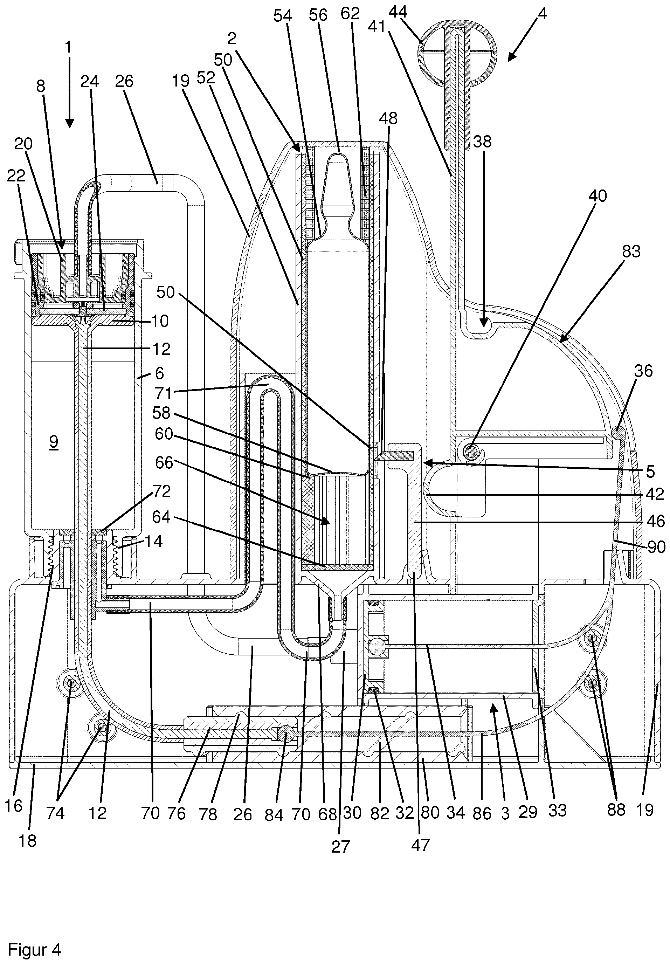

Die Kartusche 6 ist an dem Gehäuse 19 senkrecht lösbar befestigt. Die Fluidverbindung 70 mündet in dem Stutzen mit dem Außengewinde 16 durch einen pulverundurchlässigen aber für die Monomerflüssigkeit durchlässigen Filter 72 in den Innenraum der Kartusche 6. Unterhalb des Filters 72 ist ein ringförmiger Kanal 73 ausgebildet (nur in den Figuren 3 und 8 gekennzeichnet aber auch in den Figuren 4, 6 und 7 zu erkennen), der zum Filter 72 hin offen ist, so dass der ebenfalls ringförmige Filter 72 den ringförmigen Kanal abdeckt. Der ringförmige Kanal 73, in den die Fluidverbindung 70 mündet und der genaugenommen noch zur Fluidverbindung 70 gehört, und der ringförmige Filter 72 umschließen den Durchgang, in dem die Mischwelle 12 beziehungsweise das Kabel 12 in den Innenraum der Kartusche 6 geführt ist. Im Durchgang können dazu Abdichtungen (nicht gezeigt) oder zumindest Abstreifer (nicht gezeigt) vorgesehen sein. Mit dem ringförmigen Kanal 73 wird erreicht, dass die Monomerflüssigkeit durch den Filter 72 um die Mischwelle 12 herum in den Innenraum der Kartusche 6 eingeleitet wird. Es kann am Eingang der Fluidverbindung 70 in den Innenraum der Kartusche 6 auch eine Düse (nicht gezeigt) vorgesehen sein, die die Monomerflüssigkeit im Innenraum beziehungsweise im Zementpulver 9 verteilt.

Der Flüssigkeitsbehälter 2 wird mit dem Gehäuse 19 nach oben verschlossen, nachdem die Glasampulle 54 in den Flüssigkeitsbehälter 2 eingesetzt wurde. Damit die Monomerflüssigkeit ohne Probleme aus der Glasampulle 54 und dem Speichervolumen 66 auslaufen beziehungsweise ablaufen kann, können zusätzlich in dem den Flüssigkeitsbehälter 2 abdeckenden Teil des Gehäuses 19 mehrere Durchgänge (nicht gezeigt) vorgesehen sein, durch die Luft von außerhalb in den Flüssigkeitsbehälter 2 nachströmen kann. Nach dem Aufbrechen der Glasampulle 54 ist die Monomerflüssigkeit in dem Speichervolumen 66 und der Fluidverbindung 70 verfügbar und kann durch die Fluidverbindung 70 in den Innenraum der Kartusche 6 geleitet werden, in dem ein Unterdruck in dem Innenraum der Kartusche 6 verwendet wird, um die Monomerflüssigkeit aus dem Flüssigkeitsbehälter 2 in den Innenraum der Kartusche 6 zu saugen. Dieser Unterdruck wird mit der Pumpe 3 erzeugt. Im Innenraum der Kartusche 6 kann die Monomerflüssigkeit dann mit dem Zementpulver 9 mit Hilfe der Mischeinrichtung 10 unter Vakuum beziehungsweise unter Unterdruck gemischt werden, um den Knochenzement 96 beziehungsweise einen Knochenzementteig 96 zu erzeugen.

Die Mischeinrichtung 10 wird zum Durchmischen des Inhalts des Innenraums der Kartusche 6 verwendet. Das Kabel 12 beziehungsweise die Mischwelle 12, über die die Mischeinrichtung 10 im Innenraum gedreht wird und in Längsrichtung des Innenraums auf und ab bewegt wird, wird über Stifte 74 beziehungsweise Umlenkrollen 74 in Richtung eines Zylinders 76 umgelenkt. Die Umlenkrollen 74 können mit federnd gelagerten Röhrchen beziehungsweise Umlenkhülsen aufgebaut werden. Die Federn dienen dabei lediglich der Fixierung der Umlenkrollen 74 beziehungsweise Umlenkhülsen. Das Kabel 12 beziehungsweise die Mischwelle 12 ist starr mit dem Zylinder 76 verbunden. Der Zylinder 76 weist an der Außenseite ein steiles Außengewinde 78 auf. Der Zylinder 76 ist in einer Hülse 80 mit einem zum Außengewinde 78 passenden Innengewinde 82 angeordnet. Bei einer Bewegung des Zylinders 76 in der Hülse 80 in Längsrichtung (der Zylinderachse) wird die Mischeinrichtung 10 über das Kabel 12 beziehungsweise die Mischwelle 12 also in Längsrichtung des Innenraums der Kartusche 6 bewegt und gleichzeitig aufgrund der Gewinde 78, 82 um die Mischwelle 12 gedreht und damit der Inhalt des Innenraums durchmischt. Alternativ zum Außengewinde 78 an der Hülse kann auch ein oder mehrere Vorsprünge beziehungsweise eine oder mehrere Nocken 78 vorgesehen sein, die in dem Innengewinde 82 laufen und so den Zylinder 76 in der Hülse 80 drehen.

Der Zylinder 76 ist über ein Kugelgelenk beziehungsweise einen Kugelgelenkkopf 84 mit einem starren Kabel 86 oder einer flexiblen Stange 86, das analog dem Kabel 34 beziehungsweise der flexiblen Stange 34 für die Pumpe 3 aufgebaut ist, verbunden. Der Kugelgelenkkopf 84 kann sich also innerhalb einer Aufnahme für den Kugelgelenkkopf 84 des Zylinders 76 bewegen und darin drehen. Hierdurch wird ermöglicht, dass bei einer Bewegung des Kabels 86 gleichzeitig eine Drehung des Zylinders 76 in der Hülse 80 erzwungen wird. Das Kabel 86, das mit dem Zylinder 76 verbunden ist und das Kabel 34, das mit dem Kolben 30 der Pumpe 3 verbunden ist, sind miteinander verbunden, wobei beide über Stifte 88 beziehungsweise Umlenkrollen 88 positioniert werden. Die Umlenkrollen 88 sind analog den Umlenkrollen 74 aufgebaut. Ebenso kann die Verbindung des Kabels 34 zum Kolben 30 mit einem Kugelgelenk aufgebaut werden. Die beiden Kabel 34, 86 oder flexiblen Stangen 34, 86 schließen sich zu einem Kabel 90 oder einer flexiblen Stange 90 zusammen, das nach oben zum Hebel 4 beziehungsweise zum Bedienelement 4 geführt ist, wobei das Kabel 90 oder die flexible Stange 90 dort in dem Rastmittel 36 endet. Auch das Kabel 90 ist analog dem Kabel 34 für die Pumpe 3 aufgebaut, beziehungsweise die flexible Stange 90 analog der flexiblen Stange 34 für die Pumpe 3. Die gegabelt verbundenen Kabel 34, 86, 90 oder die gegabelte Stange 34, 86, 90 können aus einem Kunststoff durch Spritzguss gefertigt sein, beziehungsweise das gemeinsame gegabelte Kabel 34, 86, 90 oder die gegabelte Stange 34, 86, 90 kann aus einem Kunststoff durch Spritzguss gefertigt sein. Das Rastmittel 36 am Ende des Kabels 90 ist dabei so gelagert und derart vorgespannt, dass es in das Gegenrastmittel 38 greift und mit diesem rastet, wenn der Hebel 4 weit genug gedreht beziehungsweise geschwenkt wird, beziehungsweise wenn das Gegenrastmittel 38 auf die Höhe des Rastmittels 36 geschwenkt wird.

Der maximale Hub, der durch eine als Evolvente 83 geformte Abrundung am Hebel 4 bestimmt ist, indem das Kabel 90 oder die flexible Stange 90 nach erfolgter Rastung auf die Evolvente 83 aufgezogen wird, reicht dazu aus, dass der Innenraum der Kartusche 6 in voller Länge von der Mischeinrichtung 10 durchfahren werden kann. Dies ist in den Figuren 3, 4, 6 und 7 im Vergleich mit Figur 8 zu erkennen, da beim vollständigen Hub des Hebels 4, der in Figur 8 dargestellt ist, die Mischeinrichtung 10 beziehungsweise die Mischflügel 10 an der Vorderseite des Innenraums der Kartusche 6 auf dem Filter 72 anliegt, während ohne Hub, wie in Figur 7 dargestellt, die Mischeinrichtung 10 beziehungsweise die Mischflügel 10 auf der Rückseite des Innenraums der Kartusche 6 an dem Austragskolben 8, beziehungsweise dem Sterilisationskolben 22 und der Porenscheibe 24 anliegen. Hierdurch wird eine vollständige Durchmischung des Innenraums der Kartusche 6 mit der Mischeinrichtung 10 ermöglicht. Das Gegenrastmittel 38 ist hierzu an dem bezüglich der Zugrichtung der Kabel 90, 34, 86 oder der flexiblen Stange 90, 34, 86 abgewandten Ende der Evolvente 83 angeordnet, damit das Kabel 90 oder die flexible Stange 90 über einen weiten Bereich der Evolvente 83 aufgezogen werden kann.

Anstatt das Kabel 34 oder die flexible Stange 34 der Pumpe 3 und das Kabel 86 oder die flexible Stange 86 zum Zylinder 76 miteinander zu dem Kabel 90 oder der flexiblen Stange 90, an dem das Rastmittel 36 angeordnet ist, zu verbinden, kann genauso gut jedes der Kabel 34, 86 oder jede der flexiblen Stangen 34, 86 ein eigenes Rastmittel aufweisen, das in das Gegenrastmittel 38 oder das zwei unterschiedliche Gegenrastmittel am Ende der Evolvente 83 beziehungsweise dem Hebel 4 greift und mit diesem beziehungsweise diesen rastet.

In einer alternativen Ausführung einer erfindungsgemäßen Mischvorrichtung kann das Kabel 12 direkt mit dem Kabel 86 verbunden sein, beziehungsweise die beiden Kabel 12, 86 als ein gemeinsames durchgehendes Kabel ausgeführt sein, oder die flexible Stange 12 mit der flexiblen Stange 86 einteilig ausgeführt sein. Der Zylinder 76, die Hülse 80 sowie die Gewinde 78, 82 sind dann überflüssig und nicht vorhanden. Dies führt dann dazu, dass die Mischeinrichtung 10 nicht mehr durch die Mischwelle 12 im Innenraum der Kartusche 6 gedreht wird. Eine Durchmischung des Innenraums der Kartusche 6 wird dann nur noch durch die Auf- und Abwärtsbewegung der Mischeinrichtung 10 in Längsrichtung erreicht. Durch eine geeignete Neigung der Mischflügel 10 oder einiger Mischflügel 10 und/oder durch eine Führung zumindest eines Vorsprungs (nicht gezeigt) an der Mischeinrichtung 10 in wenigstens einer spiralförmigen Nut (nicht gezeigt) in der Innenwand der Kartusche 6 kann auch auf andere Weise eine Drehung der Mischeinrichtung 10 in der Kartusche 6 erzwungen werden, sofern nicht einfach auf die Drehung der Mischeinrichtung 10 in der Kartusche 6 verzichtet wird.

Figur 5 zeigt eine schematische Querschnittansicht der Vakuummischvorrichtung nach den Figuren 1 bis 4 und 6 bis 8 mit einer Schnittebene senkrecht zu dem Schnitt der Figuren 3 und 4 sowie 6 bis 8. Darin ist unter anderem ein beispielhafter Aufbau der Rückschlagventile 27, 28 erläutert. Die Rückschlagventile 27, 28 sind mit Kugeln 91 aufgebaut, die von Federn 92 auf einen Kugelsitz gedrückt werden, in dem sich die Verbindung zur Vakuumleitung 26 beziehungsweise zur Umgebung der Pumpe 3 befindet. Wenn die Kugeln 91 auf den Kugelsitz gedrückt sind, sind diese Verbindungen geschlossen. Das Rückschlagventil 27 öffnet sich, wenn in der Pumpe 3 ein Unterdruck relativ zum Druck in der Vakuumleitung 26 beziehungsweise relativ zum Innenraum der Kartusche 6 entsteht beziehungsweise vorhanden ist, indem der Kolben 30 in Richtung des Verschlusses 33 bewegt wird, also wenn sich der Pumpraum 94 (siehe Figur 8) öffnet. Das Rückschlagventil 28 öffnet sich dagegen, wenn im Pumpraum 94 beziehungsweise in der Pumpe 3 ein größerer Druck herrscht als in der Umgebung. Ansonsten schließen die Rückschlagventile 27, 28 aufgrund der Federkraft der Federn 92.

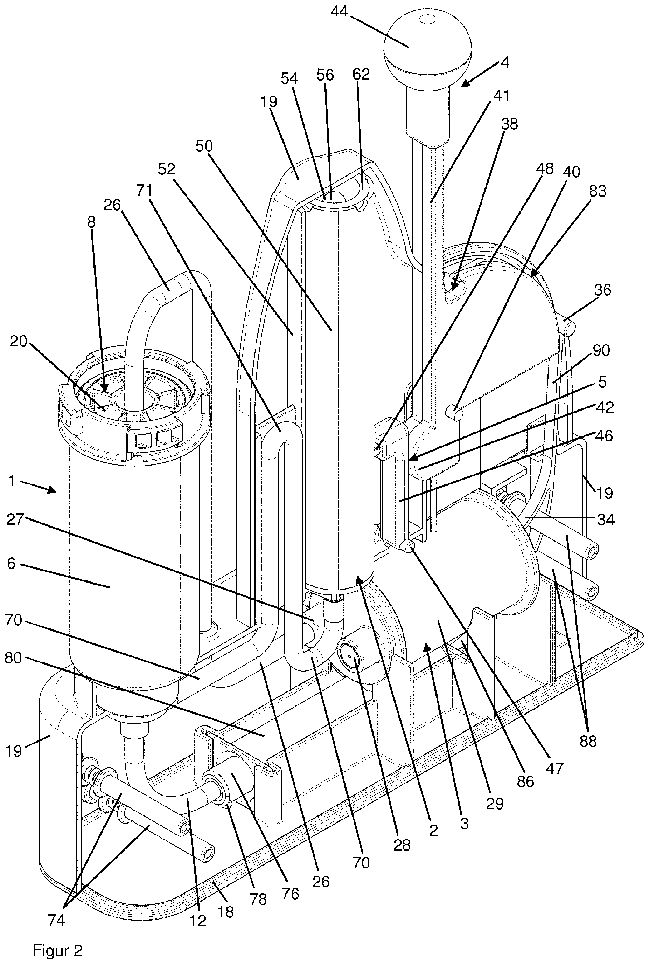

Die Vakuummischvorrichtung zeichnet sich erfindungsgemäß durch die Anwendbarkeit des folgenden beispielhaften erfindungsgemäßen Verfahrens aus. Die Monomerflüssigkeit wird in dem Flüssigkeitsbehälter 2 bereitgestellt, indem die Glasampulle 54 mit der Öffnungseinrichtung 5, wie oben geschildert, aufgebrochen wird. Dazu wird der Hebel 4, der sich ursprünglich in einer senkrechten Position (siehe Figur 1 bis 4) befindet nach unten gedrückt (siehe Figur 6). Während die Monomerflüssigkeit ausläuft und das Speichervolumen 66 und die Fluidverbindung 70 füllt, ohne über die Schlaufe 71 überzutreten, wird der Hebel 40 weiter gedreht beziehungsweise geschwenkt, bis das Gegenrastmittel 38 auf die Höhe des Rastmittels 36 gedreht ist und beide miteinander rasten (siehe Figur 7). Durch das Rasten des Rastmittels 36 mit dem Gegenrastmittel 38 kann erst jetzt die Pumpe 3 mit dem Hebel 4 über die Kabel 34, 90 oder die flexible Stange 34, 90 bewegt beziehungsweise angetrieben werden und die Mischeinrichtung 10 mit dem Hebel 4 über die Kabel 86, 90 oder die Stange 86, 90, den Zylinder 76 und das Kabel 12 beziehungsweise die Mischwelle 12 angetrieben werden. Dazu wird der Hebel 4 vom unteren Anschlag weg wieder in die Ausgangsposition (Figuren 2 bis 4) gedreht beziehungsweise geschwenkt.

Die Pumpe 3 wird verwendet, indem der Kolben 30 mit dem Bedienelement 4 über das Kabel 34, 90 oder die flexible Stange 34, 90 von den Rückschlagventilen 27, 28 weg in Richtung des Verschlusses 33 gezogen wird. Dabei öffnet sich im Inneren der Pumpe 3 der Pumpraum 94 (siehe Figur 8). Durch die Vergrößerung des Pumpraums 94 wird im Pumpraum 94 ein geringer Druck erzeugt. Dies führt zu einer Öffnung des Rückschlagventils 27, so dass Gas aus dem Innenraum der Kartusche 6 durch die Verbindungsleitung 26 in den Pumpraum 94 gesaugt beziehungsweise gedrückt wird. Aufgrund des so entstehenden Unterdrucks wird die Monomerflüssigkeit aus dem Speichervolumen 66 durch die Fluidverbindung 70 in den Innenraum der Kartusche 6 gesaugt.

Der Kolben 30 wird bis zum Ende des Hohlzylinders 29 (in den Figuren 3 bis 4 und 6 bis 8 rechts) bewegt. Diese Anordnung ist in Figur 8 gezeigt. Die Volumenzunahme des Pumpraums 96 kann vorzugsweise dazu ausreichen, um das Gas aus der Vakuumleitung 26, dem Innenraum der Kartusche 6 und der Fluidverbindung 70 zu evakuieren und die Monomerflüssigkeit aus dem Flüssigkeitsbehälter 2 in den Innenraum der Kartusche 6 zu ziehen, so dass bereits ein Hub des Kolbens 30 ausreicht, um die Monomerflüssigkeit in den Innenraum der Kartusche 6 zu transferieren. Bevorzugt kann der expandierte Pumpraum 96 zu diesem Zweck größer als die Volumina der Leitungen 26, 70, des Innenraums der Kartusche 6 und des Flüssigkeitsvolumens der Monomerflüssigkeit. Durch den Unterdruck in dem Innenraum der Kartusche 6 wird die Monomerflüssigkeit aus der Aufnahme 2 beziehungsweise dem Speichervolumen 66 durch die Fluidverbindung 70 über die Schlaufe 71 hinweg in den Innenraum der Kartusche 6 gesaugt. Alternativ zur Überführung der Monomerflüssigkeit mit einem einzigen Hub des Kolbens 30 kann die Monomerflüssigkeit aber auch mit mehreren Hüben des Kolbens 30 durch wiederholtes Bedienen des Hebels 4 (hin und her schwenken des Hebels 4) in den Innenraum der Kartusche 6 gesaugt werden.

Der Teil des Innenraums des Hohlzylinders 29 zwischen dem vorderen Verschluss mit den Rückschlagventilen 27, 28 daran (in den Figuren 3, 4 und 6 bis 8 links und in Figur 5 oben) und dem Kolben 30 bildet den Pumpraum 94. Ein Unterdruck im Pumpraum 94 kann damit durch die Vakuumleitung 26 bis in den Innenraum der Kartusche 6 wirken, beziehungsweise ein Gas aus dem Innenraum der Kartusche 6 evakuiert werden, wenn der Dichtungskolben 20, wie in den Figuren gezeigt, mit dem Sterilisationskolben 22 verbunden ist und der Innenraum der Kartusche 6 dadurch nach außen bis auf die Öffnung zur Vakuumleitung 26 abgedichtet ist.

Gleichzeitig mit der Bewegung des Kolbens 30 wird über die Kabel 86, 90 oder die Stange 86, 90 und das Kugelgelenk 84 der Zylinder 76 in der Hülse 80 in Längsrichtung bewegt und dabei über die Gewinde 78, 82 gedreht. Die Bewegung in Längsrichtung und die Drehung wird über die Mischwelle 12 beziehungsweise das Kabel 12 durch die Durchführung auf die Mischeinrichtung 10 im Innenraum der Kartusche 6 übertragen. Durch mehrfaches Schwenken des Hebels 4 in beide Richtungen und damit Bewegen der Mischeinrichtung 10 im Innenraum der Kartusche 6 wird der Inhalt, nämlich das Knochenzementpulver 9 und die eingesaugte Monomerflüssigkeit, durchmischt und dadurch ein Knochenzementteig 96 im Innenraum der Kartusche 6 erzeugt.

Wenn die Ausgangskomponenten im Innenraum der Kartusche 6 mit den Mischflügeln 10 gemischt wurden, wird das Kartuschensystem 1 von dem Gehäuse 19 beziehungsweise dem Außengewinde 16 des Gehäuses 19 abgeschraubt und das Kabel 12 beziehungsweise die Mischwelle 12 mit der Mischeinrichtung 10 aus dem Innenraum der Kartusche 6 herausgezogen. Dabei klappen die Mischflügel 10 nach oben zusammen. Hierfür sind an der Verbindung der Mischflügel 10 zu der Mischwelle 12 Material-Verjüngungen als Sollknickstellen vorgesehen.

Der Dichtungskolben 20 wird gegen den Sterilisationskolben 22 gedreht und somit die Gasdurchführung durch den Dichtungskolben 20 verschlossen. Die Vakuumleitung 26 wird vom Dichtungskolben 20 abgezogen. Nachdem das Kartuschensystem 1 abgeschraubt wurde, wird in das Innengewinde 14 ein Austragsrohr (nicht gezeigt) mit einem passenden Außengewinde eingeschraubt, durch das der gemischte Knochenzement 96 appliziert werden kann. Der aus dem Sterilisationskolben 22 und dem Dichtungskolben 20 zusammengesetzte Förderkolben 8 oder Austragskolben 8 wird entrastet und kann mit einem Applikationsgerät (nicht gezeigt) ins Innere der Kartusche 6 getrieben werden. Dadurch wird der Inhalt der Kartusche 6, also der unter Unterdruck gemischte Knochenzementteig 96 aus der gegenüberliegenden Öffnung und durch das aufgeschraubte Austragsrohr ausgepresst.

Die Bauteile der Vakuummischvorrichtung können bis auf die Glasampulle 54, die Filter 64, 72 und die Ausgangskomponenten des Knochenzements durch Spritzgießen aus Kunststoff gefertigt werden. Die Fluidverbindung 70, 71 kann aus einem anderen Kunststoff bestehen. Die Verbindungsleitung 26 kann flexibel sein, um sie leichter von dem Dichtungskolben 20 abziehen zu können.

Die Leitungen 26, 70 und die Kabel 34, 86, 90 oder die gegabelte Stange 34, 86, 90 sind in dem Gehäuse 19 aus Kunststoff angeordnet, das mit dem Fußteil 18 fest verbunden ist, wobei das Fußteil 18 einen flachen Boden aufweist, damit die Vakuummischvorrichtung auf einer flachen Unterlage aufgestellt werden kann.

Anstatt der mit dem beschriebenen Ausführungsbeispiel verwendeten Glasampulle 54 kann auch ein anderer Monomerflüssigkeitsbehälter verwendet werden. Beispielsweise kann ein Folienbeutel enthaltend die Monomerflüssigkeit als Behälter für die Monomerflüssigkeit in eine modifizierte Aufnahme eingesetzt werden. Der Folienbeutel kann beispielsweise ein mit Aluminium beschichteter Kunststoffbeutel sein, der gegen die Monomerflüssigkeit chemisch ausreichend beständig ist. In der alternativen Aufnahme 2 kann dann ein Dorn oder besser eine Klinge vorgesehen sein, der oder die mit der Öffnungseinrichtung 5 gegen den Folienbeutel zu drücken und bewegen ist, so dass der Folienbeutel über die Öffnungseinrichtung 5 mit dem Dorn oder der Klinge aufzustechen oder aufzuschlitzen ist, so dass anschließend die Monomerflüssigkeit aus dem Folienbeutel ausläuft und in der Aufnahme 2 zur Verfügung steht. Ferner kann der Behälter für die Monomerflüssigkeit auch fest in der Aufnahme 2 und damit in die Vakuummischvorrichtung integriert sein und sich mit der Öffnungseinrichtung 5 zu dem Filter 64 und/oder Sieb 64 beziehungsweise zur Fluidverbindung 70 hin öffnen lassen.

Die Variante mit Glasampulle 54 als Behälter für die Monomerflüssigkeit ist aber erfindungsgemäß bevorzugt, da die mit Monomerflüssigkeit gefüllten Glasampullen 54 kommerziell kostengünstig zu erhalten sind und zudem Glasampullen 54 zur langfristigen Lagerung der Monomerflüssigkeit besonders gut geeignet sind. Dabei ist es besonders bevorzugt, dass die Glasampulle 54 bereits in der Aufnahme 2 der Vakuummischvorrichtung enthalten ist.

Mit der beschriebenen Vakuummischvorrichtung können die beiden Ausgangskomponenten des Knochenzements gelagert und zu einem beliebigen späteren Zeitpunkt unter Vakuum gemischt werden. Dabei muss die Vakuummischvorrichtung an keine externe Versorgung (Strom, Wasser oder Druckgas) angeschlossen werden. Es ist kein interner Energiespeicher, wie eine Batterie, eine Druckgaspatrone oder eine gespannte Feder, zum Antreiben der Vakuummischvorrichtung beziehungsweise der Pumpe 3, der Mischeinrichtung 10 und der Öffnungseinrichtung 5 notwendig. Die zum Erzeugen des Unterdrucks notwendige Energie wird ebenso manuell aufgebracht, wie die zum Öffnen der Glasampulle 54 und die zum Bewegen der Mischeinrichtung 10 notwendige Kraft.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartuschensystem
- 2: Aufnahme
- 3: Pumpe
- 4: Bedienelement / Hebel
- 5: Öffnungseinrichtung
- 6: Kartusche / Kartuschenwand / Hohlzylinder
- 8: Austragskolben
- 9: Zementpulver
- 10: Mischeinrichtung / Mischflügel
- 12: Mischwelle / Kabel
- 14: Innengewinde
- 16: Außengewinde
- 18: Fußteil / Standfuß
- 19: Gehäuse
- 20: Dichtungskolben
- 22: Sterilisationskolben
- 24: Porenscheibe
- 26: Verbindungsleitung / Vakuumleitung
- 27: Rückschlagventil
- 28: Rückschlagventil / Abluft
- 29: Hohlzylinder
- 30: Kolben
- 32: Dichtung / O-Ring
- 33: Verschluss
- 34: Kabel / flexible Stange
- 36: Rastmittel
- 38: Gegenrastmittel
- 40: Achse
- 41: Hebelarm
- 42: Hebelarm
- 44: Griff
- 46: Hebel
- 47: Achse
- 48: Kante
- 50: Elastischer Einsatz
- 52: Hohlzylinder
- 54: Glasampulle mit Monomerflüssigkeit
- 56: Ampullenkopf
- 58: Ampullenboden
- 60: Auflage / Absatz
- 62: Hohlzylinder
- 64: Filter / Sieb
- 66: Speichervolumen
- 68: Trichter
- 70: Fluidverbindung / Flüssigkeitsleitung
- 71: Schlaufe
- 72: Pulverundurchlässiger und Flüssigkeitsdurchlässiger Filter
- 73: Ringförmiger Kanal
- 74: Stift / Umlenkrolle
- 76: Zylinder
- 78: Außengewinde / Nocke
- 80: Hülse
- 82: Innengewinde
- 83: Evolvente
- 84: Kugelgelenkkopf
- 86: Kabel / flexible Stange
- 88: Stift / Umlenkrolle
- 90: Kabel / flexible Stange
- 91: Kugel
- 92: Feder
- 94: Pumpraum
- 96: Knochenzementteig

## Patentansprüche

1. Vakuummischvorrichtung zum Mischen von Polymethylmethacrylat-Knochenzement aus einer Monomerflüssigkeit und einem Zementpulver (9), die Vakuummischvorrichtung aufweisend
zumindest eine Kartusche (6) umfassend einen evakuierbaren Innenraum zum Mischen des Knochenzements (96),
eine Mischeinrichtung (10) zum Durchmischen des Inhalts des Innenraums der zumindest einen Kartusche (6), die beweglich im Innenraum angeordnet ist,
eine Aufnahme (2) zur Aufnahme eines die Monomerflüssigkeit enthaltenden separaten Behälters (54) oder aufweisend einen integrierten Behälter enthaltend die Monomerflüssigkeit,
eine Öffnungseinrichtung (5), die im Bereich der Aufnahme (2) gegen die Aufnahme (2) beweglich angeordnet ist, so dass durch eine Bewegung der Öffnungseinrichtung (5) ein in der Aufnahme (2) angeordneter separater Behälter (54) mit der Öffnungseinrichtung (5) zu öffnen ist, oder die Öffnungseinrichtung (5) im Bereich des integrierten Behälters gegen den integrierten Behälter beweglich angeordnet ist, so dass durch eine Bewegung der Öffnungseinrichtung (5) der integrierte Behälter mit der Öffnungseinrichtung (5) zu öffnen ist,
eine Pumpe (3), in der ein beweglicher Kolben (30) zum Erzeugen eines Unterdrucks angeordnet ist und der einen Pumpraum (94) der Pumpe (3) begrenzt, und
eine Verbindungsleitung (26), die den Innenraum der zumindest einen Kartusche (6) mit dem Pumpraum (94) der Pumpe (3) verbindet, wobei die Vakuummischvorrichtung ein von außen bedienbares Bedienelement (4) aufweist, wobei mit dem Bedienelement (4) der Kolben (30) in der Pumpe (3) manuell bewegbar ist, und wobei
mit demselben Bedienelement (4) die Öffnungseinrichtung (5) gegen die Aufnahme (2) oder gegen den integrierten Behälter zu bewegen ist, und
mit demselben Bedienelement (4) die Mischeinrichtung (10) im Innenraum der Kartusche (6) zum Durchmischen des Inhalts des Innenraums der Kartusche (6) bewegbar ist.

2. Vakuummischvorrichtung nach Anspruch 1, **dadurch gekennzeichnet, dass** das Bedienelement (4) mit dem Kolben (30) derart verbunden oder verbindbar ist, dass der Kolben (30) in der Pumpe (3) durch Bedienen des Bedienelements (4) manuell bewegbar ist.

3. Vakuummischvorrichtung nach einem der vorangehenden Ansprüche, **dadurch gekennzeichnet, dass**
die Aufnahme (2) zumindest bereichsweise geschlossene Seitenwände (50, 52) zur Aufnahme einer Glasampulle (54) als separaten Behälter (54) aufweist, wobei die Aufnahme (2) zumindest eine verformbare geschlossene Seitenwand (50) aufweist und gegenüber der verformbaren Seitenwand (50) ein Stützelement vorgesehen ist, wobei die Öffnungseinrichtung (5) über das Bedienelement (4) gegen die verformbare Seitenwand (50) der Aufnahme (2) zu pressen ist, so dass sich die verformbare Seitenwand (50) derart verformt, dass eine in der Aufnahme (2) angeordnete passende Glasampulle (54) mit der Öffnungseinrichtung (5) aufzubrechen ist.

4. Vakuummischvorrichtung nach einem der vorangehenden Ansprüche, **dadurch gekennzeichnet, dass**
die Öffnungseinrichtung (5) einen ersten Hebel (46) aufweist, der um eine erste Achse (47) drehbar gegen die Aufnahme (2) oder den integrierten Behälter gelagert ist, wobei ein freies Ende (48) des ersten Hebels (46) gegen eine verformbare Seitenwand (50) der Aufnahme (2) oder den integrierten Behälter drückbar ist, wobei das Bedienelement (4) durch einen zweiten Hebel (4) gebildet ist, der um eine zweite Achse (40) drehbar gegen die Aufnahme (2) oder den integrierten Behälter gelagert ist, wobei die zweite Achse (40) den zweiten Hebel (4) in einen kurzen Hebelarm (42) und einen langen Hebelarm (41) teilt, wobei ein Ende des kurzen Hebelarms (42) durch manuelles Bedienen des langen Hebelarms (41) gegen den ersten Hebel (46) zu drücken ist, so dass das freie Ende des ersten Hebels (46) gegen die verformbare Seitenwand (50) drückt und diese derart verformt, dass ein in der Aufnahme (2) befindlicher separater Behälter (54) zu öffnen ist, oder das freie Ende des ersten Hebels (46) gegen den integrierten Behälter drückt, so dass der integrierte Behälter sich zu einer Fluidverbindung (70) hin öffnet.

5. Vakuummischvorrichtung nach einem der vorangehenden Ansprüche, **dadurch gekennzeichnet, dass**
das Bedienelement (4) manuell beweglich ist, vorzugsweise ein um eine Achse (40) schwenkbarer Hebel (4) ist, wobei das Bedienelement (4) derart mit der Öffnungseinrichtung (5), der Pumpe (3) und der Mischeinrichtung (10) in Wirkverbindung steht oder in Wirkverbindung zu bringen ist, dass bei einem ersten Bedienen des Bedienelements (4) ein separater Behälter (54) in der Aufnahme (2) oder der integrierte Behälter zu öffnen ist und bei einem weiteren Bedienen des Bedienelements (4) der Kolben (30) in der Pumpe (3) anzutreiben ist und die Mischeinrichtung (10) im Innenraum anzutreiben ist.

6. Vakuummischvorrichtung nach Anspruch 5, **dadurch gekennzeichnet, dass** der Kolben (30) der Pumpe (3) und/oder die Mischeinrichtung (10) über ein flexibles Kabel (34, 86, 90) und/oder eine Stange anzutreiben sind, wobei an dem flexiblen Kabel (34, 86, 90) und/oder der Stange ein Rastmittel (36) vorgesehen ist, das nach erstmaligem Bedienen des Bedienelements (4) in ein Gegenrastmittel (38) am Bedienelement (4) oder in ein mit dem Bedienelement (4) verbundenes Gegenrastmittel (38) greift, so dass bei einem der Rastung nachfolgenden Bedienen des Bedienelements (4) der Kolben (30) der Pumpe (3) und/oder die Mischeinrichtung (10) über das Kabel (34, 86, 90) und/oder die Stange mit dem Bedienelement (4) anzutreiben sind.

7. Vakuummischvorrichtung nach einem der vorangehenden Ansprüche, **dadurch gekennzeichnet, dass**
die Mischeinrichtung (10) durch Bedienen des Bedienelements (4) in dem Innenraum axial in Längsrichtung beweglich ist.

8. Vakuummischvorrichtung nach einem der vorangehenden Ansprüche, **dadurch gekennzeichnet, dass**
der Pumpraum (94) der Pumpe (3) gasdicht ist und im Inneren der Pumpe (3) angeordnet ist, wobei der Kolben (30) über das Bedienelement (4) manuell in zumindest einer Richtung antreibbar ist, so dass durch die Bewegung des Kolbens (30) der Pumpraum (94) zu vergrößern ist und mit dem dadurch im Pumpraum (94) entstehenden Unterdruck der Innenraum der zumindest einen Kartusche (6) durch die Verbindungsleitung (26) evakuierbar ist.

9. Vakuummischvorrichtung nach einem der vorangehenden Ansprüche, **dadurch gekennzeichnet, dass**
in der Verbindung vom Pumpraum (94) zu der Verbindungsleitung (26) oder in der Verbindungsleitung (26) ein erstes Einwegventil (27) angeordnet ist, das offen ist oder öffnet, wenn im Pumpraum (94) ein geringerer Druck herrscht als im Innenraum der Kartusche (6), und geschlossen ist oder schließt, wenn im Pumpraum (94) ein gleicher oder ein höherer Druck herrscht als im Innenraum der Kartusche (6), und bevorzugt ein zweites Einwegventil (28) den Pumpraum (94) mit der Umgebung der Pumpe (3) verbindet, das offen ist oder öffnet, wenn im Pumpraum (94) ein höherer Druck herrscht als in der Umgebung der Pumpe (3), und geschlossen ist oder schließt, wenn im Pumpraum (94) ein gleicher oder ein geringerer Druck herrscht als in der Umgebung der Pumpe (3).

10. Vakuummischvorrichtung nach einem der vorangehenden Ansprüche, **dadurch gekennzeichnet, dass**
der Kolben (30) über eine Stange (34, 90) und/oder ein Kabel (34, 90) mit dem Bedienelement (4) verbunden oder verbindbar ist und vorzugsweise der Kolben (30) durch Bedienen des Bedienelements (4) in der Pumpe (3) zu bewegen ist.

11. Vakuummischvorrichtung nach einem der vorangehenden Ansprüche, **dadurch gekennzeichnet, dass**
die Kartusche (6) eine mit Zementpulver (9) gefüllte Zementkartusche (6) ist und in der Aufnahme (2) ein separater Behälter (54) enthaltend eine Monomerflüssigkeit angeordnet ist oder in dem integrierten Behälter eine Monomerflüssigkeit enthalten ist, wobei vorzugsweise die Aufnahme (2) oder der integrierte Behälter durch ein zu öffnendes Trennelement flüssigkeitsundurchlässig mit dem Innenraum der Zementkartusche (6) verbunden ist und/oder der Innenraum der Zementkartusche (6) mit der Pumpe (3) gasdurchlässig verbunden ist oder verbindbar ist.

12. Vakuummischvorrichtung nach einem der vorangehenden Ansprüche, **dadurch gekennzeichnet, dass**
die Kartusche (6), die Pumpe (3) und alle Leitungen (26, 70) sowie die Aufnahme (2) oder der integrierte Behälter mit einem gemeinsamen Fußteil (18) und/oder einem Gehäuse (19) fest und/oder lösbar verbunden sind, wobei vorzugsweise die Pumpe (3) und alle Leitungen (26, 70) sowie die Aufnahme (2) oder der separate Behälter (54) fest mit dem Fußteil (18) und/oder einem Gehäuse (19) verbunden sind und die Kartusche (6) lösbar mit dem Fußteil (18) und/oder einem Gehäuse (19) verbunden ist.

13. Vakuummischvorrichtung nach einem der vorangehenden Ansprüche, **dadurch gekennzeichnet, dass**
die Aufnahme (2) oder der integrierte Behälter über eine Fluidverbindung (70) mit dem Innenraum der Kartusche (6) verbunden oder verbindbar ist, wobei bevorzugt die Einmündung der Fluidverbindung (70) in den Innenraum der Kartusche (6) auf der gegenüberliegenden Seite der Einmündung der Verbindungsleitung (26) zwischen dem Innenraum und dem Pumpraum (94) angeordnet ist.

14. Vakuummischvorrichtung nach einem der vorangehenden Ansprüche, **dadurch gekennzeichnet, dass**
durch die Bewegung des Kolbens (30) in der Pumpe (3) im Pumpraum (94) ein Unterdruck zu erzeugen ist, wobei mit dem Unterdruck ein Gas durch die Verbindungsleitung (26) aus dem Innenraum der zumindest einen Kartusche (6) evakuierbar ist.

15. Vakuummischvorrichtung nach einem der vorangehenden Ansprüche, **dadurch gekennzeichnet, dass**
die Pumpe (3) aufgebaut ist mit
einem Hohlzylinder (29), wobei der Hohlzylinder (29) mit dem Innenraum der Kartusche (6) verbunden oder verbindbar ist,
einem gasdichten Verschluss an einem Hohlzylinderende,
dem Kolben (30), der im Hohlzylinder (29) gasdicht, axial beweglich angeordnet ist, wobei
der Kolben (30) in der Pumpe (3) mit dem manuell bedienbaren Bedienelement (4) bewegbar ist, wobei
bei einem Bewegen des Kolbens (30) mit dem manuell bedienbaren Bedienelement (4) der Kolben (30) axial entgegengesetzt zum Verschluss bewegbar ist und dadurch Gas aus dem Innenraum der Kartusche (6) evakuiert, wobei
das Bedienelement (4) mit der Öffnungseinrichtung (5) in Wirkverbindung steht und wobei
das Bedienelement (4) mit der Mischeinrichtung (10) im Innenraum der Kartusche (6) derart verbunden ist, dass bei einem Bedienen des Bedienelements (4) die Mischeinrichtung (10) im Innenraum der Kartusche (6) bewegbar ist.

16. Vakuummischvorrichtung nach einem der vorangehenden Ansprüche, **dadurch gekennzeichnet, dass**
die Pumpe (3), die Öffnungseinrichtung (5) und die Mischeinrichtung (10) über die Bewegung des Bedienelements (4) antreibbar sind, wobei bevorzugt die Bewegung des Bedienelements (4) durch manuelle Krafteinwirkung erfolgt.

17. Verfahren zur Vermischung von Polymethylmethacrylat-Knochenzement in einem Innenraum einer Kartusche (6) einer Vakuummischvorrichtung, insbesondere einer Vakuummischvorrichtung nach einem der vorangehenden Ansprüche, wobei ein Bedienelement (4) bedient wird und dadurch ein integrierter Behälter der Vakuummischvorrichtung oder ein separater Behälter (54), der in einer Aufnahme (2) der Vakuummischvorrichtung angeordnet ist, geöffnet wird, wobei anschließend eine in dem integrierten Behälter oder dem separaten Behälter (54) enthaltene Monomerflüssigkeit als erste Komponente des Knochenzements (96) ausläuft,
durch eine anschließende weitere Bedienung des Bedienelements (4) eine Bewegung eines Kolbens (30) einer Pumpe (3) der Vakuummischvorrichtung angetrieben wird, wobei durch die Bewegung des Kolbens (30) ein Unterdruck in einem Pumpraum (94) der Pumpe (3) erzeugt wird, wobei mit der derart angetriebenen Pumpe (3) der Innenraum der Kartusche (6) evakuiert wird und mit dem Unterdruck im Innenraum der Kartusche (6) die Monomerflüssigkeit in den Innenraum der Kartusche (6) geleitet wird, wobei sich in dem Innenraum der Kartusche (6) bereits ein Knochenzementpulver (9) als zweite Komponente des Knochenzements (96) befindet, und
durch die Bedienung des Bedienelements (4) eine Mischeinrichtung (10) im Innenraum der Kartusche (6) bewegt wird und durch die Bewegung der Mischeinrichtung (10) ein Knochenzementteig (96) im Innenraum der Kartusche (6) aus dem Zementpulver (9) und der Monomerflüssigkeit gemischt wird.

18. Verfahren nach Anspruch 17, **dadurch gekennzeichnet, dass** der Kolben (30) der Pumpe (3) mit dem Bedienelement (4) bewegt wird, wodurch ein Unterdruck gegenüber der umgebenden Atmosphäre in der Pumpe (3) erzeugt wird,
dabei durch eine Verbindungsleitung (26) Gas aus dem Innenraum der Kartusche (6) in den Pumpraum (94) der Pumpe (3) gesaugt wird, anschließend durch Bedienen desselben Bedienelements (4) die Mischeinrichtung (10) im Innenraum der Kartusche (6) bewegt wird und dabei das Zementpulver (9) mit der Monomerflüssigkeit vermischt wird, anschließend die Kartusche (6) mit dem gemischten Zementteig (96) entnommen wird und
durch axiale Bewegung eines Austragskolbens (8) der Zementteig (96) aus der Kartusche (6) heraus gepresst wird.

19. Verfahren nach einem der Ansprüche 17 oder 18, **dadurch gekennzeichnet, dass**
das Zementpulver (9) in der Kartusche (6) angeordnet ist,
die Monomerflüssigkeit in einer von der Kartusche (6) separaten Aufnahme (2) angeordnet ist, wobei die Monomerflüssigkeit in einem integrierten Behälter oder in einem separaten Behälter (54), vorzugsweise in einer Glasampulle (54) in der Aufnahme (2), enthalten ist,
der integrierte Behälter oder der separate Behälter (54) durch Bedienen des Bedienelements (4) und einer daraus resultierenden Bewegung der Öffnungseinrichtung (5) geöffnet wird, bevor der Kolben (30) durch ein weiteres Bedienen des Bedienelements (4) angetrieben wird,
danach der Kolben (30) axial in einem Hohlzylinder (29) bewegt wird, wodurch ein Unterdruck gegenüber der umgebenden Atmosphäre erzeugt wird,
dabei durch die Verbindungsleitung (26) Gas aus dem Innenraum der Kartusche (6) in den Hohlzylinder (29) gesaugt wird und durch den in dem Innenraum der Kartusche (6) gebildeten Unterdruck Monomerflüssigkeit in die Kartusche (6) gesaugt wird.

## Claims

1. Vacuum mixing device for the mixing of polymethylmethacrylate bone cement from a monomer liquid and a cement powder (9), whereby the vacuum mixing device comprises
at least one cartridge (6) comprising an internal space, which can be evacuated, for the mixing of the bone cement (96);
a mixing facility (10) for mixing the content of the internal space of the at least one cartridge (6) that is arranged such as to be mobile in the internal space; a receptacle (2) for accommodating a separate container (54) that contains the monomer liquid or comprising an integrated container containing the monomer liquid;
an opening facility (5) that is arranged in the area of the receptacle (2) such as to be mobile with respect to the receptacle (2) such that a motion of the opening facility (5) allows a separate container (54) arranged in the receptacle (2) to be opened by the opening facility (5), or the opening facility (5) is arranged in the area of the integrated container such as to be mobile with respect to the integrated container such that a motion of the opening facility (5) allows the integrated container to be opened by the opening facility (5);
a pump (3), in which a mobile vacuum plunger (30) for generation of a negative pressure is arranged and which limits a pump space (94) of the pump (3);
a pressure pump (3), in which a mobile pump plunger (31) for conveying a liquid is arranged and limits a pressure pump space (36) of the pressure pump (3);
a connecting line (26) that connects the internal space of the at least one cartridge (6) to the pump space (94) of the pump (3); whereby the vacuum mixing device comprises an operating element (4) that can be operated from outside, whereby the plunger (30) can be moved manually in the pump (3) by means of the operating element (4); and whereby the opening facility (5) can be moved against the receptacle (2) or against the integrated container by means of the same operating element (4), and
the mixing facility (10) can be moved in the internal space of the cartridge (6) by means of the same operating element (4) in order to mix the content of the internal space of the cartridge (6).

2. Vacuum mixing device according to claim 1, **characterised in that** the operating element (4) is or can be connected appropriately to the plunger (30) such that the plunger (30) can be moved manually in the vacuum pump (3) by operating the operating element (4).

3. Vacuum mixing device according to any one of the preceding claims, **characterised in that**
the receptacle (2) comprises side walls (50, 52), at least regions of which are closed, for accommodation of a glass ampoule (54) as separate container (54), whereby the receptacle (2) comprises at least one deformable closed side wall (50) and a support element is provided opposite from the deformable side wall (50), whereby the opening facility (5) can be pressed against the deformable side wall (50) of the receptacle (2) by means of the operating element (4), such that the deformable side wall (50) is deformed appropriately such that a fitting glass ampoule (54) arranged in the receptacle (2) can be broken open by means of the opening facility (5).

4. Vacuum mixing device according to any one of the preceding claims, **characterised in that**
the opening facility (5) comprises a first lever (46) that is supported against the receptacle (2) or the integrated container such that it can rotate about a first axis (47), whereby a free end (48) of the first lever (46) can be pushed against a deformable side wall (50) of the receptacle (2) or the integrated container, whereby the operating element (4) is formed by a second lever (4) that is supported against the receptacle (2) or the integrated container such that it can rotate about a second axis (40), whereby the second axis (40) divides the second lever (4) into a short lever arm (42) and a long lever arm (41), whereby one end of the short lever arm (42) can be pushed against the first lever (46) by manual operation of the long lever arm (41) such that the free end of the first lever (46) pushes against the deformable side wall (50) and deforms said side wall appropriately such that a separate container (54) that is situated in the receptacle (2) can be opened, or the free end of the first lever (46) pushes against the integrated container such that the integrated container opens toward a fluid connection (70).

5. Vacuum mixing device according to any one of the preceding claims, **characterised in that**
the operating element (4) can be moved manually, preferably it is a lever (4) that can be swivelled about an axis (40), whereby the operating element (4) is or can be brought into an operative connection to the opening facility (5), the pump (3), and the mixing facility (10) in appropriate manner such that a separate container (54) in the receptacle (2) or the integrated container can be opened by means of a first operation of the operating element (4), and, by means of another operation of the operating element (4), the plunger (30) can be driven in the pump (3) and the mixing facility (10) can be driven in the internal space.

6. Vacuum mixing device according to claim 5, **characterised in that** the plunger (30) of the pump (3) and/or the mixing facility (10) can be driven via a flexible cable (34, 86, 90) and/or a rod, whereby the flexible cable (34, 86, 90) and/or the rod have a snap-in means (36) provided on them, which, after the operating element (4) is operated for the first time, engages an opposite snap-in means (38) on the operating element (4) or an opposite snap-in means (38) that is connected to the operating element (4) such that an operation of the operating element (4) after the snapping-in has taken place can drive the plunger (30) of the pump (3) and/or the mixing facility (10) via the cable (34, 86, 90) and/or the rod by means of the operating element (4).

7. Vacuum mixing device according to any one of the preceding claims, **characterised in that**
the mixing device is axially mobile in longitudinal direction in the internal space by operation of the operating element (4).

8. Vacuum mixing device according to any one of the preceding claims, **characterised in that**
the pump space (94) of the pump (3) is gas-tight and is arranged on the inside of the pump (3), whereby the plunger (30) can be driven manually in at least one direction by means of the operating element (4) such that the motion of the plunger (30) allows the pump space (94) to be increased and the negative pressure thus generated in the pump space (94) allows the internal space of the at least one cartridge (6) to be evacuated through the connecting line (26).

9. Vacuum mixing device according to any one of the preceding claims, **characterised in that**
a first one-way valve (27) is arranged in the connection from the pump space (94) to the connecting line (26) or in the connecting line (26), and is open or opens up, when the pressure in the pump space (94) is lower than the pressure in the internal space of the cartridge (6), and is closed or closes, when the pressure in the pump space (94) is equal to or higher than the pressure in the internal space of the cartridge (6), and, preferably, a second one-way valve (28) connects the pump space (94) to the surroundings of the pump (3) and is open or opens up, when the pressure in the pump space (94) is higher than the pressure in the surroundings of the pump (3), and is closed or closes, when the pressure in the pump space (94) is equal to or lower than the pressure in the surroundings of the pump (3).

10. Vacuum mixing device according to any one of the preceding claims, **characterised in that**
the plunger (30) is or can be connected to the operating element (4) by means of a rod (34, 90) and/or a cable (34, 90), and, preferably, the plunger (30) can be moved in the pump (3) by operation of the operating element (4).

11. Vacuum mixing device according to any one of the preceding claims, **characterised in that**
the cartridge (6) is a cement powder (9)-filled cement cartridge (6) and a separate container (54) containing a monomer liquid is arranged in the receptacle (2) or a monomer liquid is contained in the integrated container, whereby, preferably, the receptacle (2) or the integrated container is connected in liquid-impermeable manner to the internal space of the cement cartridge (6) by means of a separating element that can be opened and/or the internal space of the cement cartridge (6) is or can be connected to the pump (3) in gas-permeable manner.

12. Vacuum mixing device according to any one of the preceding claims, **characterised in that**
the cartridge (6), the pump (3), and all lines (26, 70) as well as the receptacle (2) or the integrated container are connected, firmly and/or detachably, to a common foot part (18) and/or a casing (19), whereby, preferably, the pump (3) and all lines (26, 70) as well as the receptacle (2) or the separate container (54) are firmly connected to the foot part (18) and/or a casing (19), and the cartridge (6) is detachably connected to the foot part (18) and/or a casing (19).

13. Vacuum mixing device according to any one of the preceding claims, **characterised in that**
the receptacle (2) or the integrated container is or can be connected to the internal space of the cartridge (6) by means of a fluid connection (70), whereby the merging point of the fluid connection (70) into the internal space of the cartridge (6) opposite of the merging point of the connecting line (26) between the internal space and the pump space (94).

14. Vacuum mixing device according to any one of the preceding claims, **characterised in that**
a negative pressure can be generated in the pump space (94) by moving the plunger (30) in the pump (3), whereby the negative pressure can be used to evacuate a gas through the connecting line (26) from the internal space of the at least one cartridge (6).

15. Vacuum mixing device according to any one of the preceding claims, **characterised in that**
the pump (3) is composed of
a hollow cylinder (29), whereby the hollow cylinder (29) is or can be connected to the internal space of the cartridge (6);
a gas-tight closure on one end of the hollow cylinder;
the plunger (30) that is arranged in liquid-tight manner in the hollow cylinder (29) such as to be axially mobile, whereby
the plunger (30) can be moved in the pump (3) by means of the manually operable operating element (4), whereby
the plunger (30) can be moved axially opposite to the closure (33) by moving the plunger (30) with the manually operable operating element (4) and, by this means, gas is evacuated from the internal space of the cartridge (6), whereby the operating element (4) is in an operative connection with the opening facility (5), and whereby
the operating element (4) is appropriately connected to the mixing facility (10) in the internal space of the cartridge (6) such that the mixing facility (10) can be moved in the internal space of the cartridge (6) by operating the operating element (4).

16. Vacuum mixing device according to any one of the preceding claims, **characterised in that**
the pump (3), the opening facility (5), and the mixing facility (10) can be driven by moving the operating element (4), whereby the operating element (4) is preferably moved by the action of a manual force.

17. Method for the mixing of polymethylmethacrylate bone cement in an internal space of a cartridge (6) of a vacuum mixing device, in particular of a vacuum mixing device according to any one of the preceding claims, whereby an operating element (4) is being operated and thereby an integrated container of the vacuum mixing device or a separate container (54), which is arranged in a receptacle (2) of the vacuum mixing device, is being opened, whereby a monomer liquid that is contained in the integrated container or the separate container (54) then flows out as first component of the bone cement (96);
a subsequent further operation of the operating element (4) drives a motion of a plunger (30) of a pump (3) of the vacuum mixing device, whereby the motion of the plunger (30) generates a negative pressure in a pump space (94) of the pump (3), whereby the pump (3) driven as described is used to evacuate the internal space of the cartridge (6) and the negative pressure in the internal space of the cartridge (6) is used to guide the monomer liquid into the internal space of the cartridge (6), whereby a bone cement powder (9) is already present in the internal space of the cartridge (6) as second component of the bone cement (96); and
the operation of the operating element (4) moves a mixing facility (10) in the internal space of the cartridge (6), and the motion of the mixing facility (10) mixes a bone cement dough (96) in the internal space of the cartridge (6) from the cement powder (9) and the monomer liquid.

18. Method according to claim 17, **characterised in that** the plunger (30) of the pump (3) is moved by means of the operating element (4), by means of which a negative pressure with respect to the surrounding atmosphere is generated in the pump (3);
in the process, gas from the internal space of the cartridge (6) is aspirated through a connecting line (26) into the pump space (94) of the pump (3); then the mixing facility (10) is moved in the internal space of the cartridge (6) by operation of the same operating element (4) and, in the process, the cement powder (9) is being mixed with the monomer liquid;
then the cartridge (6) containing the mixed cement dough (96) is being removed; and
the cement dough (96) is being pressed from the cartridge (6) by axial motion of a dispensing plunger (8).

19. Method according to any one of the claims 17 or 18, **characterised in that** the cement powder (9) is arranged in the cartridge (6);
the monomer liquid is arranged in a receptacle (2) that is separate from the cartridge (6), whereby the monomer liquid is contained in an integrated container or in a separate container (54), preferably is contained in a glass ampoule (54) in the receptacle (2);
the integrated container or the separate container (54) is being opened by operation of the operating element (4) and an ensuing motion of the opening facility (5), before the plunger (30) is driven by means of a further operation of the operating element (4);
then the plunger (30) is moved axially in a hollow cylinder (29), by means of which a negative pressure with respect to the surrounding atmosphere is generated;
in the process, gas is aspirated from the internal space of the cartridge (6) through the connecting line (26) into the hollow cylinder (29) and the negative pressure produced in the internal space of the cartridge (6) is used to aspirate monomer liquid into the cartridge (6).

## Revendications

1. Dispositif de mélange sous vide destiné à mélanger du ciment osseux à base de polyméthacrylate de méthyle à partir d'un monomère liquide et d'une poudre de ciment (9), le dispositif de mélange sous vide comportant au moins une cartouche (6) comportant un espace intérieur évacuable pour mélanger le ciment osseux (96),
un dispositif de mélange (10) pour mélanger le contenu de l'espace intérieur de l'au moins une cartouche (6), qui est agencée de manière mobile dans l'espace intérieur,
un logement (2) pour la réception d'un récipient séparé (54) contenant le monomère liquide ou comportant un récipient intégré contenant le monomère liquide,
un dispositif d'ouverture (5) agencé dans la zone du logement (2) de manière mobile contre le logement (2), de sorte qu'un mouvement du dispositif d'ouverture (5) permette d'ouvrir un récipient séparé (54) agencé dans le logement (2) à l'aide du dispositif d'ouverture (5), ou que le dispositif d'ouverture (5) soit agencé de manière mobile dans la zone du récipient intégré contre le récipient intégré, de sorte qu'un mouvement du dispositif d'ouverture (5) permette d'ouvrir le récipient intégré à l'aide du dispositif d'ouverture (5),
une pompe (3) qui comporte à l'intérieur un piston mobile (30) qui est destiné à la génération d'une dépression et qui délimite une chambre de pompage (94) de la pompe (3), et
une conduite de liaison (26), qui relie l'espace intérieur de l'au moins une cartouche (6) à la chambre de pompage (94) de la pompe (3), de sorte que le dispositif de mélange sous vide présente un élément de commande (4) accessible de l'extérieur, de sorte
que ledit élément de commande (4) permette de bouger manuellement le piston (30) dans la pompe (3), et de sorte
qu'il soit possible avec le même élément de commande (4) de mettre en mouvement le dispositif d'ouverture (5) contre le logement (2) ou contre le récipient intégré, et qu'il soit possible avec le même élément de commande (4) de mettre en mouvement le dispositif de mélange (10) dans l'espace intérieur de la cartouche (6) pour mélanger le contenu de l'espace intérieur de la cartouche (6).

2. Dispositif de mélange sous vide selon la revendication 1, **caractérisé en ce, que** l'élément de commande (4) soit relié ou reliable au piston (30) de telle sorte, que le piston (30) dans la pompe (3) puisse être mis en mouvement par l'actionnement de l'élément de commande (4).

3. Dispositif de mélange sous vide selon l'une des revendications précédentes, **caractérisé en ce, que**
le logement (2) présente au moins partiellement des parois latérales fermées (50, 52) pour la réception d'une ampoule en verre (54) en tant que récipient séparé (54), le logement (2) comportant au moins une paroi latérale fermée déformable (50) et un élément de support étant prévu en face de la paroi latérale déformable (50), le dispositif d'ouverture (5) pouvant être pressé via l'élément de commande (4) contre la paroi latérale déformable (50) du logement (2), de sorte que la paroi latérale déformable (50) soit déformée de telle façon qu'une ampoule en verre (54) adaptée et agencée dans le logement (2) puisse être ouverte à l'aide du dispositif d'ouverture (5).

4. Dispositif de mélange sous vide selon l'une des revendications précédentes, **caractérisé en ce, que**
le dispositif d'ouverture (5) comporte un premier levier (46) tournant autour d'un premier axe (47) contre le logement (2) ou le récipient intégré, une extrémité libre (48) du premier levier (46) pouvant être poussée contre une paroi latérale déformable (50) du logement (2) ou contre le récipient intégré, l'élément de commande (4) étant formé par un deuxième levier (4) tournant autour d'un deuxième axe (40) contre le logement (2) ou le récipient intégré, le deuxième axe (40) divisant le deuxième levier (4) en un bras de levier court (42) et un bras de levier long (41), une extrémité du bras de levier court (42) pouvant être poussée contre le premier levier (46) par l'actionnement manuel du bras de levier long (41), de sorte que l'extrémité libre du premier levier (46) exerce une pression contre la paroi latérale déformable (50) en déformant celle-ci, de sorte à générer l'ouverture d'un récipient séparé (54) logé dans le logement (2), ou que l'extrémité libre du premier levier (46) exerce une pression contre le récipient intégré, de sorte que le récipient intégré s'ouvre sur une communication fluidique (70).

5. Dispositif de mélange sous vide selon l'une des revendications précédentes, **caractérisé en ce, que**
l'élément de commande (4) puisse être mis en mouvement manuellement, qu'il soit de préférence un levier (4) pivotant autour d'un axe (40), l'élément de commande (4) étant en liaison fonctionnelle ou pouvant être mis en liaison fonctionnelle avec le dispositif d'ouverture (5), la pompe (3) et le dispositif de mélange (10), de sorte qu'un premier actionnement de l'élément de commande (4) permette d'ouvrir un récipient séparé (54) dans le logement (2) ou le récipient intégré et qu'un autre actionnement de l'élément de commande (4) permette d'entraîner le piston (30) dans la pompe (3) et d'entraîner le dispositif de mélange (10) dans l'espace intérieur.

6. Dispositif de mélange sous vide selon la revendication 5, **caractérisé en ce, que** le piston (30) de la pompe (3) et/ou le dispositif de mélange (10) puissent être actionnés par l'intermédiaire d'un câble flexible (34, 86, 90) et/ou d'une barre, un moyen de crantage (36) étant prévu au câble flexible (34, 86, 90) et/ou à la barre, de sorte que ledit moyen de crantage (36) entre en prise après le premier actionnement de l'élément de commande (4) avec un contre-moyen de crantage (38) à l'élément de commande (4) ou avec un contre-moyen de crantage (38) lié à l'élément de commande (4), de sorte que, suite au crantage, un actionnement de l'élément de commande (4) permette d'entraîner le piston (30) de la pompe (3) et/ou le dispositif de mélange (10) par l'intermédiaire du câble (34, 86, 90) et/ou de la barre avec l'élément de commande (4).

7. Dispositif de mélange sous vide selon l'une des revendications précédentes, **caractérisé en ce, que**
le dispositif de mélange (10) puisse être mis en mouvement axialement dans le sens longitudinal dans l'espace intérieur par l'actionnement de l'élément de commande (4).

8. Dispositif de mélange sous vide selon l'une des revendications précédentes, **caractérisé en ce, que**
la chambre de pompage (94) de la pompe (3) soit étanche aux gaz et qu'elle soit agencée à l'intérieur de la pompe (3), le piston (30) pouvant être entraîné manuellement dans au moins un sens par l'intermédiaire de l'élément de commande (4), de sorte que le mouvement du piston (30) permette d'agrandir la chambre de pompage (94) et que la dépression ainsi produite dans la chambre de pompage (94) permette d'évacuer l'espace intérieur de l'au moins une cartouche (6) via la conduite de liaison (26).

9. Dispositif de mélange sous vide selon l'une des revendications précédentes, **caractérisé en ce, que**
soit agencé dans la liaison de la chambre de pompage (94) à la conduite de liaison (26) ou dans la conduite de liaison (26) un premier clapet anti-retour (27), qui est ouvert ou qui ouvre lorsque la pression dans la chambre de pompage (94) est inférieure à celle dans l'espace intérieur de la cartouche (6), et qui est fermé ou qui ferme lorsque la pression dans la chambre de pompage (94) est identique ou supérieure à celle dans l'espace intérieur de la cartouche (6), et que , de préférence, la chambre de pompage (94) soit reliée à l'environnement de la pompe (3) par un deuxième clapet anti-retour (28), qui est ouvert ou qui ouvre lorsque la pression dans la chambre de pompage (94) est supérieure à celle dans l'environnement de la pompe (3), et qui est fermé ou qui ferme lorsque la pression dans la chambre de pompage (94) est identique ou inférieure à celle dans l'environnement de la pompe (3).

10. Dispositif de mélange sous vide selon l'une des revendications précédentes, **caractérisé en ce, que**
le piston (30) soit relié ou puisse être relié à l'élément de commande (4) par l'intermédiaire d'une barre (34, 90) et/ou d'un câble (34, 90) et que, de préférence, le piston (30) puisse être mis en mouvement dans la pompe (3) par l'actionnement de l'élément de commande (4).

11. Dispositif de mélange sous vide selon l'une des revendications précédentes, **caractérisé en ce, que**
la cartouche (6) soit une cartouche de ciment (6) remplie de poudre de ciment (9) et que soit agencé dans le logement (2) un récipient séparé (54) contenant un monomère liquide ou que le récipient intégré contienne un monomère liquide, le logement (2) ou le récipient intégré étant de préférence relié via un élément de séparation ouvrable de manière imperméable aux liquides avec l'espace intérieur de la cartouche de ciment (6) et/ou l'espace intérieur de la cartouche de ciment (6) étant relié ou reliable de manière perméable aux gaz à la pompe (3).

12. Dispositif de mélange sous vide selon l'une des revendications précédentes, **caractérisé en ce, que**
la cartouche (6), la pompe (3) et toutes les conduites (26, 70) ainsi que le logement (2) ou le récipient intégré soient reliés de manière fixe ou démontable avec un socle (18) commun et/ou avec un boitier (19), la pompe (3) et toutes les conduites (26, 70) ainsi que le logement (2) ou le récipient séparé (54) étant de préférence reliés de manière fixe avec le socle (18) et/ou avec un boitier (19) et la cartouche (6) de manière démontable avec le socle (18) et/ou avec un boitier (19).

13. Dispositif de mélange sous vide selon l'une des revendications précédentes, **caractérisé en ce, que**
le logement (2) ou le récipient intégré soit relié ou reliable avec l'espace intérieur de la cartouche (6) par l'intermédiaire d'une communication fluidique (70), l'embouchure de la communication fluidique (70) débouchant dans l'espace intérieur de la cartouche (6) étant de préférence agencée sur le côté opposé de l'embouchure de la conduite de liaison (26) entre l'espace intérieur et la chambre de pompage (94).

14. Dispositif de mélange sous vide selon l'une des revendications précédentes, **caractérisé en ce, que**
la mise en mouvement du piston (30) dans la pompe (3) permette de générer dans la chambre de pompage (94) une dépression, qui permet d'évacuer de l'espace intérieur de l'au moins une cartouche (6) un gaz à travers la conduite de liaison (26).

15. Dispositif de mélange sous vide selon l'une des revendications précédentes, **caractérisé en ce, que**
la structure de la pompe (3) comporte
un cylindre creux (29), ce cylindre creux (29) étant relié ou pouvant être relié à l'espace intérieur de la cartouche (6),
un système de fermeture étanche aux gaz à une extrémité du cylindre creux, le piston (30), qui est agencé de manière étanche aux gaz avec une mobilité axiale dans le cylindre creux (29), de sorte,
que le piston (30) dans la pompe (3) soit déplaçable par l'intermédiaire de l'élément de commande (4) pouvant être actionné manuellement, de sorte, que, lors d'un déplacement du piston (30) à l'aide de l'élément de commande (4) manoeuvrable manuellement, le piston (30) soit déplaçable axialement dans le sens opposé à la fermeture et qu'il évacue ainsi du gaz de l'espace intérieur de la cartouche (6), de sorte,
que l'élément de commande (4) soit en liaison fonctionnelle avec le dispositif d'ouverture (5) et de sorte,
que l'élément de commande (4) soit relié dans l'espace intérieur de la cartouche (6) avec le dispositif de mélange (10) de telle façon, que le dispositif de mélange (10) soit déplaçable dans l'espace intérieur de la cartouche (6) lors de l'actionnement de l'élément de commande (4).

16. Dispositif de mélange sous vide selon l'une des revendications précédentes, **caractérisé en ce, que**
la pompe (3), le dispositif d'ouverture (5) et le dispositif de mélange (10) puissent être entraînés par le mouvement de l'élément de commande (4), le mouvement de l'élément de commande (4) étant de préférence engendré par un actionnement manuel.

17. Procédé destiné au mélange de ciment osseux à base de polyméthacrylate de méthyle dans un espace intérieur d'une cartouche (6) d'un dispositif de mélange sous vide, notamment un dispositif de mélange sous vide selon l'une des revendications précédentes, **caractérisé en ce,**
**qu'**un élément de commande (4) soit actionné et que cette action entraîne l'ouverture d'un récipient intégré du dispositif de mélange sous vide ou d'un récipient séparé (54) agencé dans un logement (2) du dispositif de mélange sous vide, un monomère liquide contenu dans le récipient intégré ou dans le récipient séparé (54) s'écoulant ensuite en tant que premier composant du ciment osseux (96),
**que** par un autre actionnement subséquent de l'élément de commande (4), un piston (30) d'une pompe (3) du dispositif de mélange sous vide soit entraîné, le mouvement du piston (30) générant une dépression dans une chambre de pompage (94) de la pompe (3), de sorte que la pompe (3) entraînée ainsi génère l'évacuation de l'espace intérieur de la cartouche (6) et que la dépression ainsi générée dans l'espace intérieur de la cartouche (6) entraîne l'acheminement du monomère liquide dans l'espace intérieur de la cartouche (6), l'espace intérieur de la cartouche (6) contenant déjà une poudre de ciment osseux (9) en tant que deuxième composant du ciment osseux (96), et
**que** l'actionnement de l'élément de commande (4) génère un mouvement d'un dispositif de mélange (10) dans l'espace intérieur de la cartouche (6) et que ce mouvement du dispositif de mélange (10) génère le mélange d'une pâte de ciment osseux (96) dans l'espace intérieur de la cartouche (6) à partir de la poudre de ciment (9) et du monomère liquide.

18. Procédé selon la revendication 17, **caractérisé en ce, que** le piston (30) de la pompe (3) soit mis en mouvement à l'aide de l'élément de commande (4), ce qui génère une dépression dans la pompe (3) par rapport à l'atmosphère ambiante,
du gaz étant aspiré à travers une conduite de liaison (26) de l'espace intérieur de la cartouche (6) dans la chambre de pompage (94) de la pompe (3) pendant cette opération,
qu'ensuite, le dispositif de mélange (10) soit mis en mouvement dans l'espace intérieur de la cartouche (6) par l'actionnement du même élément de commande (4), ce qui entraîne le mélange de la poudre de ciment (9) avec le monomère liquide,
qu'ensuite, la cartouche (6) avec la pâte de ciment (96) soit retirée et que la pâte de ciment (96) soit pressée hors de la cartouche (6) par le mouvement axial d'un piston d'extraction (8).

19. Procédé selon l'une des revendications 17 ou 18, **caractérisé en ce, que** la poudre de ciment (9) soit agencée dans la cartouche (6),
le monomère liquide soit agencé dans un logement (2) séparé de la cartouche (6), le monomère liquide étant contenu dans un récipient intégré ou dans un récipient séparé (54), de préférence dans une ampoule en verre (54) dans le logement (2),
le récipient intégré ou le récipient séparé (54) soit ouvert par l'actionnement de l'élément de commande (4) et par le mouvement du dispositif d'ouverture (5) qui en résulte, avant que le piston (30) soit entraîné par un autre actionnement de l'élément de commande (4),
qu'ensuite le piston (30) soit mis en mouvement axialement dans un cylindre creux (29), ce mouvement générant une dépression par rapport à l'atmosphère ambiante,
ce qui génère l'aspiration à travers la conduite de liaison (26) du gaz de l'espace intérieur de la cartouche (6) dans le cylindre creux (29) ainsi que la formation d'une dépression dans l'espace intérieur de la cartouche (6), ladite dépression générant une aspiration du monomère liquide dans la cartouche (6).
